# EUROPÄISCHE PATENTSCHRIFT

(11) **EP 3 271 421 B1**
(45) Veröffentlichungstag und Bekanntmachung des Hinweises auf die Patenterteilung: **21.11.2018**
(21) Anmeldenummer: 16711208.5
(22) Anmeldetag: 16.03.2016
(51) Int. Cl.: C08L 83/04, C09D 183/04, C09J 183/04

(54) **HÄRTBARE SILIKONZUSAMMENSETZUNGEN**
CURABLE SILICON COMPOSITIONS
COMPOSITIONS DE SILICONE DURCISSABLES

(30) Priorität: 17.03.2015 DE 102015204787
(43) Veröffentlichungstag der Anmeldung: 24.01.2018
(73) Patentinhaber: Henkel AG & Co. KGaA, 40589 Düsseldorf (DE)
(72) Erfinder: GUTACKER, Andrea, 40764 Langenfeld (DE); KLEIN, Johann, 40593 Düsseldorf (DE); BOUDET, Helene, 40721 Hilden (DE); DURACU, Adrian, 40589 Düsseldorf (DE); KAPUSTA, Sebastian, 41466 Neuss (DE)
(86) Internationale Anmeldenummer: PCT/EP2016/055620
(87) Internationale Veröffentlichungsnummer: WO 2016/146648

(56) Entgegenhaltungen:
- EP-A1- 2 030 976
- WO-A1-2013/022532

## Beschreibung

Die Erfindung betrifft härtbare Zusammensetzungen auf Basis eines härtbaren Polyorganosiloxans, die neben dem Polyorganosiloxan einen speziellen Silanvernetzer, ein Aminosilan, sowie als Härtungskatalysator eine Zinnverbindung enthalten, wobei Aminosilan und Zinnverbindung in einem definierten Mengenverhältnis eingesetzt werden. Diese Zusammensetzungen zeichnen sich insbesondere durch eine ausgezeichnete Lagerstabilität aus. Weiterhin betrifft die Erfindung ein Verfahren zur Herstellung der härtbaren Zusammensetzungen, sowie deren Verwendung.

Silikonpolymere (Polyorganosiloxane), insbesondere Polydialkylsiloxane wie Polydimethylsiloxan (PDMS), haben große Bedeutung bei der Herstellung von Kleb-, Dicht-, Beschichtungs- und Isolierstoffen. Unter diesen machen solche, die bei niedrigen Temperaturen und unter Umgebungsbedingungen vulkanisieren, einen nicht unerheblichen Marktanteil aus. Typische Formulierungen enthalten ein reaktives Polyorganosiloxan. Dabei handelt es sich in der Regel um ein Silanol-terminiertes Polyorganosiloxan, wobei das Polyorganosiloxan mindestens eine, vorzugsweise zwei, an ein Siliziumatom gebundene Hydroxygruppen aufweist. Dieses wird in Kombination mit einem Vernetzer auf Silanbasis eingesetzt, der an das Siliziumatom gebundene hydrolysierbare Gruppen aufweist. Statt von Vernetzer wird gelegentlich auch von Härter gesprochen. Im Sinne dieser Anmeldung sind die Begriffe Vernetzer und Härter gleichbedeutend. Das Polyorganosiloxan und der Vernetzer können als separate Komponenten vorliegen. Oftmals wird jedoch das Polyorganosiloxan gezielt mit dem Vernetzer zu einem modifizierten Polyorganosiloxan umgesetzt, und dieses modifizierte Polyorganosiloxan der härtbaren Zusammensetzung zugegeben. Man spricht in diesem Zusammenhang auch von Endgruppenverkappung (endcapping). Diese kann gegebenenfalls in Gegenwart eines Katalysators durchgeführt werden, wobei dieser selektiv die Endgruppenverkappung vermitteln soll ohne gleichzeitig das Polyorganosiloxan zu härten.

Es sind zahlreiche Vernetzer für Silikonsysteme bekannt. Diese lassen sich anhand der bei der Hydrolyse freigesetzten Austrittsgruppen in saure, basische und neutrale Vernetzter unterscheiden. Typische saure Vernetzer enthalten als hydrolysierbare Gruppen Säuregruppen und setzen bei der Vernetzung die korrespondierenden Säuren, z.B. Essigsäure, frei. Typische basische Vernetzer setzen bei der Vernetzung Amine frei. In beiden Fällen werden bei der Vernetzung aggressive Verbindungen freigesetzt, die z.B. Metalle, Stein oder Mörtel korrodieren oder zersetzen können, und die zudem einen intensiven, oftmals unangenehmen Geruch besitzen. Daher werden für moderne härtbare Silikonzusammensetzungen häufig neutrale Vernetzer verwendet. Typische Vertreter neutraler Vernetzer weisen hydrolysierbare Gruppen auf, die bei der Vernetzung Alkohol oder Oxim abspalten. Alkoxysysteme haben allerdings den Nachteil, dass vielfach Probleme bei der Lagerstabilität entsprechender härtbarer Zusammensetzungen auftreten und die ausgehärteten Produkte auf einigen Materialien nur schlechte Haftung aufweisen. Oximosilan-Vernetzer, die unter Abgabe eines Alkanonoxims hydrolysieren, weisen diese Nachteile in der Regel nicht auf und kommen daher vielfach zum Einsatz. Der gebräuchlichste Vertreter der Oximosilan-Vernetzer setzt beim Vernetzen Butan-2-onoxim frei. Dieses steht in Verdacht Krebs zu erzeugen, so dass dringend Bedarf an alternativen neutralen Vernetzern besteht. Im Übrigen besitzen auch die freigesetzten Oxime einen intensiven, üblen Geruch und das Arbeiten mit härtbaren Zusammensetzungen, die einen entsprechenden Vernetzer enthalten, wird von den Anwendern als unangenehm empfunden.

Als alternative Vernetzer wurden daher bereits Silanverbindungen vorgeschlagen, die beim Vernetzen α-Hydroxycarbonsäureester oder α-Hydroxycarbonsäureamide freisetzen.

Die Herstellung geeigneter Silanverbindungen ist lange bekannt und beispielsweise von M. M. Sprung in "Some α-Carbalkoxyalkoxysilanes", J. Org. Chem., 1958, 23 (10), Seiten 1530-1534, beschrieben.

Auch DE 32 10 337 A1 offenbart entsprechende Silanverbindungen, deren Herstellung und Verwendung in härtbaren Zusammensetzungen auf Basis von Polydiorganosiloxanen, die kondensationsfähige Endgruppen aufweisen.

Aus EP 2 030 976 A1 sind Härter für Silikonkautschukmassen bekannt, die drei 2-Hydroxyproprionsäurealkylester-Reste, d.h. Milchsäurealkylester-Reste, aufweisen. Besonders bevorzugt ist dabei das Vinyl-tris(ethyllactato)silan.

EP 2 774 672 A1 beschreibt spezielle Katalysatoren für die Vernetzung von Silikonkautschukmassen mit einem Vernetzer auf Basis einer Silanverbindung mit Lactatgruppen. Bei dem Vernetzer kann es sich wiederum um die aus EP 2 030 976 A1 bekannten Verbindungen handeln. Es werden jedoch auch Vernetzer offenbart, die nur einen, zwei oder auch vier 2-Hydroxyproprionsäurealkylester-Reste aufweisen.

Obwohl der Einsatz eines Vernetzers auf Basis einer Silanverbindung mit Lactatgruppen oder ähnlichen α-Carbalkoxyalkoxygruppen viele Vorteile mit sich bringt, haben sich diese Vernetzer in der Praxis noch nicht durchsetzen können. Dies liegt insbesondere an der Schwierigkeit, härtbare Zusammensetzungen auf Silikonbasis, die diese Vernetzer enthalten, so zu formulieren, dass eine hinreichende Lagerstabilität erzielt wird. Gerade in Gegenwart anderer üblicher und vielfach unverzichtbarer Bestandteile solcher Zusammensetzungen, insbesondere von Härtungskatalysatoren und Haftvermittlern, leidet die Lagerstabilität drastisch.

Es ist daher eine Aufgabe der vorliegenden Erfindung, härtbare Zusammensetzungen auf Basis von Polyorganosiloxanen zur Verfügung zu stellen, die den Einsatz von Vernetzern, die beim Vernetzen α-Hydroxycarbonsäureester oder α-Hydroxycarbonsäureamide freisetzen, erlauben, und dennoch über ausgezeichnete Lagerstabilität verfügen.

Die vorliegende Erfindung löst die Aufgabe durch Bereitstellung von härtbaren Zusammensetzungen auf Basis von Polyorganosiloxanen, wobei die Zusammensetzungen neben besagtem Vernetzer mindestens ein Aminosilan und mindestens eine Zinnverbindung enthalten, wobei das Molverhältnis von Aminosilan und Zinnverbindung in einem eng definierten Bereich liegt.

Gegenstand der Erfindung sind daher härtbare Zusammensetzungen, enthaltend
(A) mindestens ein Polyorganosiloxan, das mindestens eine, an ein Siliziumatom gebundene, Hydroxygruppe aufweist,
(B) mindestens ein Silan der Formel (1):

   Si(R¹)ₘ(R²)ₙ(R³)₄₋₍ₘ₊ₙ₎ (1)

   wobei
   jedes R¹ unabhängig steht für:
      einen substituierten oder unsubstituierten Alkyl-, Alkenyl- oder Alkinylrest;
      einen substituierten oder unsubstituierten cycloaliphatischen Rest oder Arylrest;
      einen substituierten oder unsubstituierten heteroalicyclischen Rest oder Heteroarylrest;
   jedes R² unabhängig steht für einen Rest der allgemeinen Formel (2):

      -OCR⁴₂COOR⁵ (2)
   wobei
   jedes R⁴ unabhängig steht für:
      Wasserstoff; oder
      einen substituierten oder unsubstituierten Alkyl-, Alkenyl- oder Alkinylrest;
   R⁵ steht für:
      einen substituierten oder unsubstituierten Alkyl-, Alkenyl- oder Alkinylrest;
   jedes R³ unabhängig steht für einen Rest der allgemeinen Formel (3):

      -OCR⁶₂CONR⁷R⁸ (3)
   wobei
   jedes R⁶ unabhängig steht für:
      Wasserstoff oder
      einen substituierten oder unsubstituierten Alkyl-, Alkenyl- oder Alkinylrest;
   R⁷ steht für:
      Wasserstoff,
      einen substituierten oder unsubstituierten Alkyl-, Alkenyl- oder Alkinylrest, einen substituierten oder unsubstituierten cycloaliphatischen Rest oder Arylrest, R⁸, oder
      einen Rest -(CH₂)_{q}-COOR⁹, wobei q eine ganze Zahl von 2 bis 10, insbesondere 2 ist und R⁹ für einen substituierten oder unsubstituierten Alkyl-, Alkenyl- oder Alkinylrest, oder einen substituierten oder unsubstituierten cycloaliphatischen Rest oder Arylrest steht;
   R⁸ steht für einen Rest der allgemeinen Formel (4):

      -R¹⁰-SiR¹¹ₒ(OR¹²)₃₋ₒ (4)
   wobei
   R¹⁰ steht für:
      einen, gegebenenfalls durch ein Heteroatom unterbrochenen Alkylenrest;
   jedes R¹¹ unabhängig steht für:
      einen substituierten oder unsubstituierten Alkyl-, Alkenyl- oder Alkinylrest;
   jedes R¹² unabhängig steht für:
      einen substituierten oder unsubstituierten Alkyl-, Alkenyl- oder Alkinylrest, einen Acylrest,
      oder einen Rest der Formel (5):

         -CR¹³₂COOR¹⁴ (5)
   wobei
   jedes R¹³ unabhängig steht für:
      Wasserstoff; oder
      einen substituierten oder unsubstituierten Alkyl-, Alkenyl- oder Alkinylrest;
   R¹⁴ steht für:
      einen substituierten oder unsubstituierten Alkyl-, Alkenyl- oder Alkinylrest; und
   o unabhängig für 0, 1 oder 2 steht, und
   m unabhängig für 0 oder 1 und n unabhängig für 0, 1, 2, 3 oder 4 steht, wobei die Summe n + m maximal 4 beträgt,
(C) mindestens ein Aminosilan, und
(D) mindestens eine Zinnverbindung,
wobei das Molverhältnis von Aminosilan zu Zinnverbindung 1: 1 bis 50 : 1 beträgt.

Durch Einstellen des Mengenverhältnisses von Aminosilan und Zinnverbindung im erfindungsgemäßen Bereich wird gewährleistet, dass die härtbare Zusammensetzung einerseits sehr hohe Lagerstabilität aufweist, und andererseits nach Applikation in Gegenwart von Luftfeuchtigkeit bereits bei Raumtemperatur (23°C) zuverlässig und mit hinreichender Geschwindigkeit aushärtet.

Ein weiterer Gegenstand der Erfindung ist ein Verfahren zur Herstellung der erfindungsgemäßen härtbaren Zusammensetzungen, wobei das Polyorganosiloxan, das mindestens eine, an ein Siliziumatom gebundene, Hydroxygruppe aufweist, das Silan der Formel (1), das Aminosilan, die Zinnverbindung und gegebenenfalls mindestens ein weiterer Inhaltsstoff miteinander gemischt werden.

Die Erfindung betrifft ferner die Verwendung einer erfindungsgemäßen härtbaren Zusammensetzung oder einer härtbaren Zusammensetzung hergestellt nach dem erfindungsgemäßen Verfahren als Kleb-, Dicht- oder Beschichtungsstoff.

Unter einer "härtbaren Zusammensetzung" wird ein Stoff oder eine Mischung aus mehreren Stoffen verstanden, der beziehungsweise die durch physikalische oder chemische Maßnahmen härtbar ist. Dabei können diese chemischen oder physikalischen Maßnahmen beispielsweise in der Zuführung von Energie in Form von Wärme, Licht oder sonstiger elektromagnetischer Strahlung, aber auch in einfachster Inkontaktbringung mit Luftfeuchtigkeit, Wasser oder einer reaktiven Komponente bestehen. Die Zusammensetzung geht dabei vom Ausgangszustand in einen Zustand über, der höhere Härte aufweist.

Sofern in der vorliegenden Anmeldung auf Molekulargewichte von Oligomeren oder Polymeren Bezug genommen wird, beziehen sich die Angaben, sofern nicht anders angegeben, auf das Gewichtsmittel, d.h. den M_{w}-Wert, und nicht das arithmetische Mittel. Das Molekulargewicht wird mittels Gelpermeationschromatographie (GPC) mit Tetrahydrofuran (THF) als Eluent nach DIN 55672-1:2007-08 bestimmt, vorzugsweise bei 35 °C. Molekulargewichte von monomeren Verbindungen werden unter Zugrundelegung der jeweiligen Summenformel und der bekannten Molekulargewichte der einzelnen Atome berechnet.

"Mindestens ein", wie hierin verwendet, bedeutet 1 oder mehr, d.h. 1, 2, 3, 4, 5, 6, 7, 8, 9 oder mehr. Bezogen auf einen Inhaltsstoff bezieht sich die Angabe auf die Art des Inhaltsstoffs und nicht auf die absolute Zahl der Moleküle. "Mindestens ein Polymer" bedeutet somit beispielsweise mindestens eine Art von Polymer, d.h. dass eine Art von Polymer oder eine Mischung mehrerer verschiedener Polymere verwendet werden kann. Zusammen mit Gewichtsangaben bezieht sich die Angabe auf alle Verbindungen der angegebenen Art, die in der Zusammensetzung/Mischung enthalten sind, d.h. dass die Zusammensetzung über die angegebene Menge der entsprechenden Verbindungen hinaus keine weiteren Verbindungen dieser Art enthält.

Alle Prozentangaben, die im Zusammenhang mit den hierin beschriebenen Zusammensetzungen gemacht werden, beziehen sich, sofern nicht explizit anders angegeben, auf Gew.-%, jeweils bezogen auf die betreffende Mischung.

"Alkyl", wie hierin verwendet, bezieht sich auf einen gesättigten aliphatischen Kohlenwasserstoff einschließlich geradkettiger und verzweigtkettiger Gruppen. Vorzugsweise besitzt die Alkylgruppe 1 bis 10 Kohlenstoffatome (wenn ein numerischer Bereich z.B. "1-10" hierin angegeben wird, ist gemeint, dass diese Gruppe, in diesem Fall die Alkylgruppe, 1 Kohlenstoffatom, 2 Kohlenstoffatome, 3 Kohlenstoffatome etc. bis zu einschließlich 10 Kohlenstoffatome besitzen kann). Insbesondere kann es sich bei dem Alkyl um ein mittleres Alkyl, das 5 bis 6 Kohlenstoffatome besitzt, oder ein Niederalkyl, das 1 bis 4 Kohlenstoffatome besitzt z.B. Methyl, Ethyl, n-Propyl, Isopropyl, Butyl, iso-Butyl, tert-Butyl etc., handeln. Die Alkylreste können substituiert oder unsubstituiert sein. "Substituiert", wie in diesem Zusammenhang verwendet, bedeutet, dass ein oder mehrere Kohlenstoffatom(e) und/oder Wasserstoffatom(e) des Alkylrests durch Heteroatome oder funktionelle Gruppen ersetzt sind. Durch das Ersetzen eines oder mehrerer Kohlenstoffatome durch Heteroatome werden Heteroalkylgruppen erhalten, in denen 1 oder mehrere Kohlenstoffatome durch Heteroatome, insbesondere ausgewählt aus O, S, N und Si, ersetzt sind. Beispiele für solche Heteroalkylgruppen sind, ohne Einschränkung, Methoxymethyl, Ethoxyethyl, Propoxypropyl, Methoxyethyl, Isopentoxypropyl, Ethylaminoethyl, Trimethoxypropylsilyl, etc. Funktionelle Gruppen, die Wasserstoffatome ersetzen können, werden insbesondere ausgewählt aus =O, =S, -OH, -SH, -NH₂ -NO₂, -CN, -F, -Cl, -Br, -I, -OCN, -NCO, C₃-₈ Cycloalkyl, C₆-₁₄ Aryl, einem 5-10 gliedrigen Heteroarylring, in dem 1 bis 4 Ringatome unabhängig Stickstoff, Sauerstoff oder Schwefel sind, und einem 5-10 gliedrigen heteroalicyclischen Ring, in dem 1 bis 3 Ringatome unabhängig Stickstoff, Sauerstoff oder Schwefel sind.

"Alkenyl", wie hierin verwendet, bezieht sich auf eine Alkylgruppe, wie hierin definiert, die aus mindestens zwei Kohlenstoffatomen und mindestens einer Kohlenstoff-Kohlenstoff-Doppelbindung besteht, z.B. Ethenyl, Propenyl, Butenyl oder Pentenyl und deren strukturelle Isomere wie 1- oder 2-Propenyl, 1-, 2-, oder 3-Butenyl, etc. Alkenylgruppen können substituiert oder unsubstituiert sein. Wenn sie substituiert sind, sind die Substituenten wie oben für Alkyl definiert.

"Alkinyl", wie hierin verwendet, bezieht sich auf eine Alkylgruppe, wie hierin definiert, die aus mindestens zwei Kohlenstoffatomen und mindestens einer Kohlenstoff-Kohlenstoff-Dreifachbindung besteht, z. B. Ethinyl (Acetylen), Propinyl, Butinyl oder Petinyl und deren strukturelle Isomere wie oben beschrieben. Alkinylgruppen können substituiert oder unsubstituiert sein. Wenn sie substituiert sind, sind die Substituenten wie oben für Alkyl definiert.

Ein "cycloaliphatischer Rest" oder "Cycloalkylgruppe", wie hierin verwendet, bezieht sich auf monocyclische oder polycyclische (mehrere Ringe, die gemeinsame Kohlenstoffatome besitzen) Gruppen, insbesondere aus 3-8 Kohlenstoffatomen, in denen der Ring kein vollständiges konjugiertes pi-Elektronensystem besitzt, z.B. Cyclopropyl, Cyclobutyl, Cyclopentyl, Cyclohexyl, Cyclobutenyl, Cyclopentenyl, Cyclohexenyl, etc. Cycloalkylgruppen können substituiert oder unsubstituiert sein. "Substituiert", wie in diesem Zusammenhang verwendet, bedeutet, dass ein oder mehrere Wasserstoffatom(e) des Cycloalkylrests durch funktionelle Gruppen ersetzt sind. Funktionelle Gruppen, die Wasserstoffatome ersetzen können, werden insbesondere ausgewählt aus =O, =S, -OH, -SH, -NH₂ -NO₂, -CN, -F, -Cl, -Br, -I, -OCN, -NCO, C₁-₁₀ Alkyl, C₂₋₁₀ Alkenyl, C₂₋₁₀ Alkinyl, C₃-₈ Cycloalkyl, C₆-₁₄ Aryl, einem 5-10 gliedrigen Heteroarylring in dem 1 bis 4 Ringatome unabhängig Stickstoff, Sauerstoff oder Schwefel sind, und einem 5-10 gliedrigen heteroalicyclischen Ring in dem 1 bis 3 Ringatome unabhängig Stickstoff, Sauerstoff oder Schwefel sind.

"Aryl", wie hierin verwendet, bezieht sich auf monocyclische oder polycyclische (d.h. Ringe, die benachbarte Kohlenstoffatompaare gemeinsam haben) Gruppen, aus insbesondere 6 bis 14 Kohlenstoffringatomen die ein vollständiges konjugiertes pi-Elektronensystem besitzen. Beispiele für Arylgruppen sind Phenyl, Naphthalinyl und Anthracenyl. Arylgruppen können substituiert oder unsubstituiert sein. Wenn sie substituiert sind, sind die Substituenten wie oben für Cycloalkyl definiert.

Eine "Heteroaryl"-Gruppe, wie hierin verwendet, bezieht sich auf einen monocyclischen oder polycyclische (d.h. Ringe, die sich ein benachbartes Ringatompaar teilen) aromatischen Ring, aus insbesondere 5 bis 10 Ringatomen, wobei ein, zwei, drei oder vier Ringatome Stickstoff, Sauerstoff oder Schwefel sind und der Rest Kohlenstoff ist. Beispiele für Heteroarylgruppen sind Pyridyl, Pyrrolyl, Furyl, Thienyl, Imidazolyl, Oxazolyl, Isoxazolyl, Thiazolyl, Isothiazolyl, Pyrazolyl, 1,2,3-Triazolyl, 1,2,4-Triazolyl, 1,2,3-Oxadiazolyl, 1,2,4-Oxadiazolyl, 1,2,5-Oxadiazolyl, 1,3,4-Oxadiazolyl, 1,3,4-Triazinyl, 1,2,3-Triazinyl, Benzofuryl, Isobenzofuryl, Benzothienyl, Benzotriazolyl, Isobenzothienyl, Indolyl, Isoindolyl, 3H-Indolyl, Benzimidazolyl, Benzothiazolyl, Benzoxazolyl, Chinolizinyl, Chinazolinyl, Pthalazinyl, Chinoxalinyl, Cinnolinyl, Napthyridinyl, Chinolyl, Isochinolyl, Tetrazolyl, 5,6,7,8-Tetrahydrochinolyl, 5, 6, 7, 8-Tetrahydroisochinolyl, Purinyl, Pteridinyl, Pyridinyl, Pyrimidinyl, Carbazolyl, Xanthenyl oder Benzochinolyl. Heteroarylgruppen können substituiert oder unsubstituiert sein. Wenn sie substituiert sind, sind die Substituenten wie oben für Cycloalkyl definiert.

Ein "heteroalicyclischer Rest" oder eine "Heterocycloalkylgruppe", wie hierin verwendet, bezieht sich auf einen monocyclischen oder fusionierten Ring aus 5 bis 10 Ringatomen, der ein, zwei oder drei Heteroatome enthält, die aus N, O und S ausgewählt werden, wobei der Rest der Ringatome Kohlenstoff ist. Eine "Heterocycloalkenyl"-Gruppe enthält zusätzlich ein oder mehrere Doppelbindungen. Der Ring hat jedoch kein vollständiges konjugiertes pi-Elektronensystem. Beispiele für heteroalicyclische Gruppen sind Pyrrolidin, Piperidin, Piperazin, Morpholin, Imidazolidin, Tetrahydropyridazin, Tetrahydrofuran, Thiomorpholin, Tetrahydropyridin, und ähnliche. Heterocycloalkylgruppen können substituiert oder unsubstituiert sein. Wenn sie substituiert sind, sind die Substituenten wie oben für Cycloalkyl definiert.

Die erfindungsgemäßen härtbaren Zusammensetzungen enthalten als Komponente (A) mindestens ein Polyorganosiloxan, das mindestens eine, an ein Siliziumatom gebundene, Hydroxygruppe aufweist. Vorzugsweise weist das Polyorganosiloxan mindestens zwei, an ein Siliziumatom gebundene, Hydroxygruppen auf. Es ist zudem bevorzugt, dass die Hydroxygruppe oder Hydroxygruppen an terminale Siliziumatome gebunden sind. Ist das Polyorganosiloxan verzweigt, weist es bevorzugt an jedem Ende eine Hydroxygruppe auf.

Vorzugsweise handelt es sich bei dem Polyorganosiloxan, das mindestens eine, an ein Siliziumatom gebundene, Hydroxygruppe aufweist, um ein Polydiorganosiloxan, vorzugsweise ein Polydimethylsiloxan.

Bevorzugt kommt daher als Polyorganosiloxan, das mindestens eine, an ein Siliziumatom gebundene, Hydroxygruppe aufweist, ein α,ω-dihydroxyterminiertes Polydiorganosiloxan, insbesondere ein α,ω-dihydroxyterminiertes Polydimethylsiloxan zum Einsatz. Besonders bevorzugt sind α,ω-dihydroxyterminierte Polydimethylsiloxane, die eine kinematische Viskosität bei 25°C von 5000 bis 120000 cSt, insbesondere 10000 bis 100000 cSt und besonders bevorzugt 50000 bis 90000 cSt aufweisen.

Die härtbaren Zusammensetzungen enthalten das mindestens eine Polyorganosiloxan, das mindestens eine, an ein Siliziumatom gebundene, Hydroxygruppe aufweist vorzugsweise in einer Menge von 30 bis 90 Gew.-%, besonders bevorzugt in einer Menge von 40 bis 60 Gew.-%, jeweils bezogen auf das Gesamtgewicht der Zusammensetzung. Wird ein Gemisch mehrerer Polyorganosiloxane eingesetzt, beziehen sich die Mengenangaben selbstverständlich auf die Gesamtmenge an Polyorganosiloxanen, die mindestens eine, an ein Siliziumatom gebundene, Hydroxygruppe aufweisen, in der Zusammensetzung.

Die erfindungsgemäßen härtbaren Zusammensetzungen enthalten als Komponente (B) mindestens ein Silan der Formel (1):

Si(R¹)ₘ(R²)ₙ(R³)₄₋₍ₘ₊ₙ₎ (1).

Dabei steht jedes R¹ unabhängig für einen substituierten oder unsubstituierten Alkyl-, Alkenyl- oder Alkinylrest; einen substituierten oder unsubstituierten cycloaliphatischen Rest oder Arylrest; oder einen substituierten oder unsubstituierten heteroalicyclischen Rest oder Heteroarylrest.

Vorzugsweise steht jedes R¹ unabhängig voneinander für einen Alkylrest mit 1 bis 10 Kohlenstoffatomen, vorzugsweise mit 1 bis 4 Kohlenstoffatomen, insbesondere Methyl, Ethyl, Propyl oder Isopropyl, für einen Alkenylrest mit 2 bis 10 Kohlenstoffatomen, vorzugsweise 2 bis 4 Kohlenstoffatomen, insbesondere Vinyl oder Allyl, oder einen Arylrest mit 6 bis 10 Kohlenstoffatomen, insbesondere Phenyl.

Besonders bevorzugt steht R¹ unabhängig voneinander für Methyl, Vinyl oder Phenyl, ganz besonders bevorzugt für Methyl oder Vinyl.

In Formel (1) steht jedes R² unabhängig voneinander für einen Rest der allgemeinen Formel (2):

-OCR⁴₂COOR⁵ (2),

wobei
jedes R⁴ unabhängig steht für:
   Wasserstoff; oder
   einen substituierten oder unsubstituierten Alkyl-, Alkenyl- oder Alkinylrest; und
R⁵ steht für:
   einen substituierten oder unsubstituierten Alkyl-, Alkenyl- oder Alkinylrest.

Mit anderen Worten handelt es sich bei R² um einen α-Hydroxycarbonsäureester-Rest.

Vorzugsweise steht jedes R² unabhängig voneinander für einen Rest der Formel (2), wobei einer der Reste R⁴ für Wasserstoff und der zweite der Reste R⁴ für Wasserstoff oder einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, insbesondere Methyl, und R⁵ für einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, besonders bevorzugt Methyl oder Ethyl, steht.

Vorzugsweise steht jedes R² unabhängig voneinander für einen Rest der Formel (2), wobei einer der Reste R⁴ für Wasserstoff und der zweite der Reste R⁴ für Methyl, und R⁵ für Ethyl, steht.

In Formel (1) steht jedes R³ unabhängig voneinander für einen Rest der allgemeinen Formel (3):

-OCR⁶₂CONR⁷R⁸ (3).

Mit anderen Worten handelt es sich bei R³ um einen α-Hydroxycarbonsäureamid-Rest.

Dabei steht jedes R⁶ unabhängig steht für:
Wasserstoff oder
einen substituierten oder unsubstituierten Alkyl-, Alkenyl- oder Alkinylrest;
R⁷ steht für:
Wasserstoff,
einen substituierten oder unsubstituierten Alkyl-, Alkenyl- oder Alkinylrest,
einen substituierten oder unsubstituierten cycloaliphatischen Rest oder Arylrest,
R⁸, oder
einen Rest -(CH₂)_{q}-COOR⁹, wobei q eine ganze Zahl von 2 bis 10, insbesondere 2 ist und R⁹ für einen substituierten oder unsubstituierten Alkyl-, Alkenyl- oder Alkinylrest, oder einen substituierten oder unsubstituierten cycloaliphatischen Rest oder Arylrest steht;
R⁸ steht für einen Rest der allgemeinen Formel (4):

-R¹⁰-SiR¹¹ₒ(OR¹²)₃₋ₒ (4)

wobei
R¹⁰ steht für:
   einen, gegebenenfalls durch ein Heteroatom unterbrochenen Alkylenrest;
   jedes R¹¹ unabhängig steht für:
      einen substituierten oder unsubstituierten Alkyl-, Alkenyl- oder Alkinylrest;
      jedes R¹² unabhängig steht für:
         einen substituierten oder unsubstituierten Alkyl-, Alkenyl- oder Alkinylrest,
         einen Acylrest,
         oder einen Rest der Formel (5):

            -CR¹³₂COOR¹⁴ (5)
wobei
jedes R¹³ unabhängig steht für:
   Wasserstoff; oder
   einen substituierten oder unsubstituierten Alkyl-, Alkenyl- oder Alkinylrest;
R¹⁴ steht für:
   einen substituierten oder unsubstituierten Alkyl-, Alkenyl- oder Alkinylrest; und
o unabhängig für 0, 1 oder 2 steht, und
m unabhängig für 0 oder 1 und n unabhängig für 0, 1, 2, 3 oder 4 steht, wobei die Summe n + m maximal 4 beträgt.

Bevorzugt steht einer der Reste R⁶ für Wasserstoff und der zweite der Reste R⁶ für Wasserstoff oder einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, insbesondere Methyl.

R⁷ steht vorzugsweise für Wasserstoff, einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, insbesondere Methyl.

R¹⁰ ist vorzugsweise ein Alkylenrest der Formel -(CH₂)ₚ-, wobei p eine ganze Zahl von 1 bis 6, insbesondere 3 ist.

Jedes R¹¹ steht vorzugsweise unabhängig voneinander für einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, besonders bevorzugt Methyl oder Ethyl.

Jedes R¹² steht vorzugsweise unabhängig voneinander für einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, besonders bevorzugt Methyl oder Ethyl.

Jedes R¹³ steht vorzugsweise für Wasserstoff oder einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, insbesondere Methyl. Besonders bevorzugt steht ein Rest R¹³ für Wasserstoff und der zweite der Reste R¹³ für Wasserstoff oder einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, insbesondere Methyl.

R¹⁴ steht vorzugsweise für einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, besonders bevorzugt Methyl oder Ethyl.

o steht für 0, 1 oder 2, vorzugsweise für 0 oder 1, besonders bevorzugt für 0.

Vorzugsweise steht jedes R³ unabhängig voneinander für einen Rest der Formel (3), wobei einer der Reste R⁶ für Wasserstoff und der zweite der Reste R⁶ für Wasserstoff oder einen substituierten oder unsubstituierten Alkylrest mit 1 bis 10 Kohlenstoffatomen, vorzugsweise einen unsubstituierten Alkylrest mit 1 bis 10 Kohlenstoffatomen, vorzugsweise mit 1 bis 4 Kohlenstoffatomen, insbesondere Methyl, R⁷ für Wasserstoff, einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, insbesondere Methyl, und R⁸ für einen Rest der Formel (4) steht, wobei R¹⁰ ein Alkylenrest der Formel -(CH₂)ₚ- ist, wobei p eine ganze Zahl von 1 bis 6, insbesondere 3 ist, jedes R¹¹ unabhängig voneinander für einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, besonders bevorzugt Methyl oder Ethyl, und jedes R¹² unabhängig voneinander für einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, besonders bevorzugt Methyl oder Ethyl, und o für 0, 1 oder 2, vorzugsweise 0 oder 1 steht.

Besonders bevorzugt steht jedes R³ unabhängig voneinander für einen Rest der Formel (3), wobei einer der Reste R⁶ für Wasserstoff und der zweite der Reste R⁶ für Methyl, R⁷ für Wasserstoff oder Methyl, und R⁸ für einen Rest der Formel (4) steht, wobei R¹⁰ ein Alkylenrest der Formel -(CH₂)ₚ-ist, wobei p für 3 steht, jedes R¹¹ unabhängig voneinander für Methyl oder Ethyl, und jedes R¹² unabhängig voneinander für Methyl oder Ethyl, und o für 0, 1 oder 2, vorzugsweise 0 oder 1, besonders bevorzugt 0 steht.

In einer ersten Ausführungsform, sind in Formel (1) n und m so gewählt, dass die Summe n + m 4 beträgt. In diesem Fall enthält das Silan der Formel (1) keinen Rest R³, d.h. keinen α-Hydroxycarbonsäureamid-Rest. Bevorzugte Silane der Formel (1) sind in diesem Fall ausgewählt aus Methyl-tris(ethyllactato)silan, Ethyl-tris(ethyllactato)silan, Phenyl-tris(ethyllactato)silan, Vinyl-tris(ethyllactato)silan, Tetra(ethyllactato)silan und Mischungen davon.

In einer zweiten Ausführungsform, sind in Formel (1) n und m so gewählt, dass die Summe n + m maximal 3 beträgt. In diesem Fall enthält das Silan der Formel (1) mindestens einen Rest R³, d.h. mindestens einen α-Hydroxycarbonsäureamid-Rest. Bevorzugte Silane der Formel (1) sind in diesem Fall ausgewählt aus Verbindungen, die durch gezielte Amidierung von Methyl-tris(ethyllactato)silan, Ethyl-tris(ethyllactato)silan, Phenyl-tris(ethyllactato)silan, Vinyl-tris(ethyllactato)silan, Tetra(ethyllactato)silan oder Mischungen davon mit einem Amin der Formel (7):

(HR⁷N)-R¹⁰-SiR¹¹ₒ(OR¹²)₃₋ₒ (7)

erhalten werden, wobei
o, R⁷, R¹⁰, sowie jedes R¹¹ und jedes R¹², jeweils unabhängig voneinander, die oben angegebenen allgemeinen, bevorzugten und besonders bevorzugten Bedeutungen haben. Besonders bevorzugt handelt es sich um ein Amidierungsprodukt von Methyl-tris(ethyllactato)silan, Ethyl-tris(ethyllactato)silan, Phenyl-tris(ethyllactato)silan, Vinyl-tris(ethyllactato)silan, Tetra(ethyllactato)silan oder Mischungen davon mit 3-Aminopropyltrimethoxysilan und/oder 3-Aminopropyltriethoxysilan.

Die härtbaren Zusammensetzungen enthalten das Silan der Formel (1) vorzugsweise in einer Menge von 2 bis 7 Gew.-%, besonders bevorzugt in einer Menge von 4 bis 6 Gew.-%, jeweils bezogen auf das Gesamtgewicht der Zusammensetzung. Wird ein Gemisch mehrerer Silane der Formel (1) eingesetzt, beziehen sich die Mengenangaben selbstverständlich auf die Gesamtmenge an Silanen der Formel (1) in der Zusammensetzung.

Die härtbaren Zusammensetzungen können das Polyorganosiloxan, das mindestens eine, an ein Siliziumatom gebundene Hydroxygruppe aufweist, und das Silan der Formel (1) als separate Bestandteile enthalten. Es ist jedoch ebenso möglich, dass diese Bestandteile in Form eines Prepolymers vorliegen. Bei dem Prepolymer handelt es sich um ein Umsetzungsprodukt der beiden Bestandteile. Entsprechende Umsetzungen sind bekannt und werden auch als Endcapping bezeichnet. Dieses kann gegebenenfalls in Gegenwart eines Katalysators durchgeführt werden, wobei dieser selektiv die Endgruppenverkappung vermitteln soll ohne gleichzeitig das Polyorganosiloxan zu härten. Geeignete Katalysatoren sind beispielsweise Säuren, organische Lithium-Verbindungen, wie sie beispielsweise in EP 0 564 253 A1 beschrieben werden, Amine, anorganische Oxide, Kaliumacetat, Organotitanderivate, Titan/Amin-Kombinationen und Carbonsäure/Amin-Kombinationen.

Liegen das Polyorganosiloxan, das mindestens eine, an ein Siliziumatom gebundene Hydroxygruppe aufweist, und das Silan der Formel (1) als Prepolymer vor, so sind die oben genannten Mengenangaben für Polyorganosiloxan einerseits und Silan andererseits für das Prepolymer additiv anzusetzen. Die härtbaren Zusammensetzungen enthalten das Prepolymer demnach vorzugsweise in einer Menge von 32 bis 97 Gew.-%, besonders bevorzugt in einer Menge von 44 bis 66 Gew.-%, jeweils bezogen auf das Gesamtgewicht der Zusammensetzung. Wird ein Gemisch mehrerer Prepolymere eingesetzt, beziehen sich die Mengenangaben selbstverständlich auf die Gesamtmenge an Prepolymeren in der Zusammensetzung.

Die härtbaren Zusammensetzungen enthalten als Komponente (C) mindestens ein Aminosilan.

Vorzugsweise handelt es sich bei dem Aminosilan um ein Aminosilan der Formel (6),

(R¹⁵R⁷N)-R¹⁰-SiR¹¹ₒ(OR¹²)₃₋ₒ (6)

wobei
o, R⁷, R¹⁰, sowie jedes R¹¹ und jedes R¹², jeweils unabhängig voneinander, die oben angegebenen allgemeinen, bevorzugten und besonders bevorzugten Bedeutungen haben, und
R¹⁵ steht für:
   Wasserstoff,
   einen substituierten oder unsubstituierten Alkyl-, Alkenyl- oder Alkinylrest.
R¹⁵ steht vorzugsweise für Wasserstoff, einen substituierten oder unsubstituierten Alkylrest mit 1 bis 10 Kohlenstoffatomen, vorzugsweise einen unsubstituierten Alkylrest mit 1 bis 10 Kohlenstoffatomen, insbesondere mit 1 bis 4 Kohlenstoffatomen, insbesondere Methyl. Besonders bevorzugt steht R¹⁵ für Wasserstoff oder Methyl.

Das Aminosilan ist bevorzugt ausgewählt aus 3-Aminopropyltrimethoxysilan, 3-Aminopropyltriethoxysilan, Aminomethyltrimethoxysilan, Aminomethyltriethoxysilan, 3-Aminopropylmethyldiethoxysilan, (N-2-Aminoethyl)-3-aminopropyltrimethoxysilan, (N-2-Aminoethyl)-3-aminopropyltriethoxysilan, Diethylenetriaminopropyltrimethoxysilan, Phenylaminomethyltrimethoxysilan, (N-2-Aminoethyl)-3-aminopropylmethyldimethoxysilan, 3-(N-Phenylamino)propyltrimethoxysilan, 3-Piperazinylpropylmethyldimethoxysilan, 3-(N,N-Dimethylaminopropyl)-aminopropyl-methyldimethoxysilan, Tri[(3-triethoxysilyl)propyl]amin, Tri[(3-trimethoxysilyl)propyl]amin und deren Oligomere, 3-(N,N-Dimethylamino)propyltrimethoxysilan, 3-(N,N-Dimethylamino)propyltriethoxysilan, (N,N-Dimethylamino)methyltrimethoxysilan, (N,N-Dimethylamino)methyltriethoxysilan, Bis(3-trimethoxysilyl)propylamin, Bis(3-triethoxysilyl)propylamin und Mischungen davon, besonders bevorzugt aus 3-Aminopropyltrimethoxysilan, 3-Aminopropyltriethoxysilan, Aminomethyltrimethoxysilan, Aminomethyltriethoxysilan, 3-(N,N-Dimethylamino)propyltrimethoxysilan, 3-(N,N-Dimethylamino)propyltriethoxysilan, (N,N-Dimethylamino)methyltrimethoxysilan, (N,N-Dimethylamino)methyltriethoxysilan, Bis(3-trimethoxysilyl)propylamin, Bis(3-triethoxysilyl)propylamin und Mischungen davon.

Die härtbaren Zusammensetzungen enthalten das Aminosilan vorzugsweise in einer Menge von 0,05 bis 4 Gew.-%, bevorzugt in einer Menge von 0,1 bis 2 Gew.-%, besonders bevorzugt in einer Menge von 0,2 bis 2 Gew.-%, jeweils bezogen auf das Gesamtgewicht der Zusammensetzung. Wird ein Gemisch mehrerer Aminoilane eingesetzt, beziehen sich die Mengenangaben selbstverständlich auf die Gesamtmenge an Aminosilanen in der Zusammensetzung.

Die härtbaren Zusammensetzungen enthalten schließlich als Komponente (D) mindestens eine Zinnverbindung.

Vorzugsweise handelt es sich dabei um eine zinnorganische Verbindung oder ein anorganisches Zinnsalz. Zinn liegt in diesen Zinnverbindungen vorzugsweise zwei- oder vierwertig vor. Die Komponente (D) wird der Zusammensetzung insbesondere als Vernetzungskatalysator zugegeben. Geeignete anorganische Zinnsalze sind beispielsweise Zinn(II)chlorid und Zinn(IV)chlorid. Vorzugsweise kommen als Zinnverbindungen jedoch zinnorganische Verbindungen (Zinnorganyle) zum Einsatz. Geeignete zinnorganische Verbindungen sind beispielsweise die 1,3-Dicarbonylverbindungen des zwei- bzw. vierwertigen Zinns, beispielsweise die Acetylacetonate wie Di(n-butyl)zinn-(IV)-di(acetylacetonat), Di(n-octyl)zinn-(IV)-di(acetylacetonat), (n-Octyl)(n-butyl)-zinn-(IV)-di(acetylacetonat); die Dialkylzinn-(IV)-Dicarboxylate, zum Beispiel Di-n-butylzinndilaurat, Di-n-butylzinnmaleat, Di-n-butylzinndiacetat, Di-n-octylzinndilaurat Di-n-octylzinndiacetat oder die entsprechenden Dialkoxylate, zum Beispiel Di-n-butylzinndimethoxid; Oxide des vierwertigen Zinns, beispielsweise Dialkylzinnoxide, wie beispielsweise Di-n-butylzinnoxid und Di-n-octylzinnoxid; sowie die Zinn-(II)-carboxylate wie Zinn(II)octoat oder Zinn(II)phenolat.

Ferner eignen sich Zinnverbindungen von Ethylsilicat, Dimethylmaleat, Diethylmaleat, Dioctylmaleat, Dimethylphthalat, Diethylphthalat, Dioctylphthalat, wie beispielsweise Di(n-butyl)zinn(IV)-di(methylmaleat), Di(n-butyl)zinn(IV)-di(butylmaleat), Di(n-octyl)zinn(IV)-di(methylmaleat), Di(n-octyl)zinn(IV)-di(butylmaleat), Di(n-octyl)zinn(IV)-di(iso-octylmaleat); sowie Di(n-butyl)zinn(IV)sulfid, (n-Butyl)₂Sn(SCH₂COO), (n-Octyl)₂Sn(SCH₂COO), (n-Octyl)₂Sn(SCH₂CH₂COO), (n-Octyl)₂Sn(SCH₂CH₂COOCH₂CH₂OCOCH₂S), (n-Butyl)₂Sn(SCH₂COO-i-C₈H₁₇)₂, (n-Octyl)₂Sn(SCH₂COO-i-C₈H₁₇)₂, (n-Octyl)₂Sn(SCH₂COO-n-C₈H₁₇)₂.

Bevorzugt ist die Zinnverbindung ausgewählt aus 1,3-Dicarbonylverbindungen des zwei- bzw. vierwertigen Zinns, den Dialkylzinn-(IV)-Dicarboxylaten, den Dialkylzinn-(IV)-Dialkoxylaten, den Dialkylzinn-(IV)-oxiden, den Zinn-(II)-carboxylaten und Mischungen davon.

Besonders bevorzugt ist die Zinnverbindung ein Dialkylzinn-(IV)-Dicarboxylat, insbesondere Di-n-butylzinndilaurat oder Di-n-octylzinndilaurat.

Die härtbaren Zusammensetzungen enthalten die Zinnverbindung vorzugsweise in einer Menge von 0,01 bis 2 Gew.-%, bevorzugt in einer Menge von 0,05 bis 2 Gew.-%, besonders bevorzugt in einer Menge von 0,1 bis 0,5 Gew.-%, jeweils bezogen auf das Gesamtgewicht der Zusammensetzung. Wird ein Gemisch mehrerer Zinnverbindungen eingesetzt, beziehen sich die Mengenangaben selbstverständlich auf die Gesamtmenge an Zinnverbindungen in der Zusammensetzung.

Die erfindungsgemäßen Zusammensetzungen vernetzen in Gegenwart von Feuchtigkeit und härten dabei unter Ausbildung von Si-O-Si-Bindungen aus.

Durch Einstellen des Molverhältnisses von Aminosilan und Zinnverbindung im Bereich von 1 : 1 bis 50 : 1 wird dabei gewährleistet, dass die härtbare Zusammensetzung einerseits sehr hohe Lagerstabilität aufweist, und andererseits nach Applikation in Gegenwart von Luftfeuchtigkeit bereits bei Raumtemperatur (23°C) zuverlässig und mit hinreichender Geschwindigkeit aushärtet.

Vorzugsweise beträgt das Molverhältnis von Aminosilan zu Zinnverbindung 10 : 1 bis 40 : 1, besonders bevorzugt 20 : 1 bis 35 : 1.

Die härtbaren Zusammensetzungen können neben den zwingenden Bestandteilen noch ein oder mehrere weitere Bestandteile enthalten, die dazu dienen können, bestimmte Eigenschaften der härtbaren Zusammensetzung und/oder des ausgehärteten Produkts gezielt zu beeinflussen.

Diese weiteren Bestandteile können beispielsweise ausgewählt sein aus der Gruppe umfassend Weichmacher, Stabilisatoren, Antioxidantien, Füllstoffe, Reaktivverdünner, Trockenmittel, Haftvermittler, UV-Stabilisatoren, rheologische Hilfsmittel und/oder Lösungsmittel. Dabei kommt den Weichmachern, Füllstoffen und Stabilisatoren, umfassend Antioxidantien und UV-Stabilisatoren, besondere Bedeutung zu.

Bevorzugt enthalten die härtbaren Zusammensetzungen daher mindestens eine weiteren Bestandteil.

Es ist denkbar, dass die Viskosität der härtbaren Zusammensetzung für bestimmte Anwendungen zu hoch ist. Diese kann man dann in der Regel durch Verwendung eines Reaktivverdünners auf einfache und zweckmäßige Weise verringern, ohne dass es zu Entmischungserscheinungen (z.B. Weichmacherwanderung) in der ausgehärteten Masse kommt.

Vorzugsweise weist der Reaktivverdünner mindestens eine funktionelle Gruppe auf, die nach der Applikation z.B. mit Feuchtigkeit oder Luftsauerstoff reagiert. Beispiele für derartige Gruppen sind Silylgruppen, Isocyanatgruppen, vinylisch ungesättigte Gruppen und mehrfach ungesättigte Systeme.

Als Reaktivverdünner kann man alle Verbindungen, die mit den anderen Bestandteilen unter Verringerung der Viskosität mischbar sind und über mindestens eine mit dem Polymer reaktive Gruppe verfügen, einsetzen.

Die Viskosität des Reaktivverdünners beträgt bevorzugt weniger als 20.000 mPas, besonders bevorzugt etwa 0,1 - 6.000 mPas, ganz besonders bevorzugt 1 - 1000 mPas (Brookfield RVT, 23 °C, Spindel 7, 10 U/min).

Als Reaktivverdünner kann man z.B. folgende Stoffe einsetzen: mit Isocyanatosilanen umgesetzte Polyalkylenglykole (z.B. Synalox 100-50B, DOW), Carbamatopropyltrimethoxysilan, Alkyltrimethoxysilan, Alkyltriethoxysilan, wie Methyltrimethoxysilan, Methyltriethoxysilan sowie Vinyltrimethoxysilan (XL 10, Wacker), Vinyltriethoxysilan, Phenyltrimethoxysilan, Phenyltriethoxysilan, Octyltrimethoxysilan, Tetraethoxysilan, Vinyldimethoxymethylsilan (XL12, Wacker), Vinyltriethoxysilan (GF56, Wacker), Vinyltriacetoxysilan (GF62, Wacker), Isooctyltrimethoxysilan (IO Trimethoxy), Isooctyltriethoxysilan (IO Triethoxy, Wacker), N-Trimethoxysilylmethyl-O-methylcarbamat (XL63, Wacker), N-Dimethoxy(methyl)silylmethyl-O-methyl-carbamat (XL65, Wacker), Hexadecyltrimethoxysilan, 3-Octanoylthio-1-propyltriethoxysilan und Teilhydrolysate dieser Verbindungen.

Ferner sind ebenfalls folgende Polymere von Kaneka Corp. als Reaktivverdünner einsetzbar: MS S203H, MS S303H, MS SAT 010, und MS SAX 350.

Ebenso kann man silanmodifizierte Polyether verwenden, die sich z.B. aus der Umsetzung von Isocyanatosilan mit Synalox Typen ableiten.

Weiterhin kann man als Reaktivverdünner Polymere einsetzen, die aus einem organischen Grundgerüst durch Pfropfen mit einem Vinylsilan oder durch Umsetzung von Polyol, Polyisocyanat und Alkoxysilan herstellbar sind.

Unter einem Polyol wird eine Verbindung verstanden, die im Molekül eine oder mehrere OH - Gruppen enthalten kann. Die OH -Gruppen können sowohl primär als auch sekundär sein.

Zu den geeigneten aliphatischen Alkoholen zählen beispielsweise Ethylenglykol, Propylenglykol und höhere Glykole, sowie andere polyfunktionelle Alkohole. Die Polyole können zusätzlich weitere funktionelle Gruppen wie z.B. Ester, Carbonate, Amide enthalten.

Zur Herstellung der bevorzugten Reaktivverdünner wird die entsprechende Polyolkomponente jeweils mit einem mindestens difunktionellen Isocyanat umgesetzt. Als mindestens difunktionelles Isocyanat kommt grundsätzlich jedes Isocyanat mit mindestens zwei Isocyanatgruppen in Frage, in der Regel sind jedoch im Rahmen der vorliegenden Erfindung Verbindungen mit zwei bis vier Isocyanatgruppen, insbesondere mit zwei Isocyanatgruppen bevorzugt.

Vorzugsweise weist die als Reaktivverdünner vorliegende Verbindung mindestens eine Alkoxysilylgruppe auf, wobei unter den Alkoxysilylgruppen die Di- und Trialkoxysilylgruppen bevorzugt sind.

Als Polyisocyanate zur Herstellung eines Reaktivverdünners eignen sich beispielsweise Ethylendiisocyanat, 1,4-Tetramethylendiisocyanat, 1,4-Tetramethoxybutandiisocyanat, 1,6-Hexamethylendiisocyanat (HDI), Cyclobutan-1,3-diisocyanat, Cyclohexan-1,3- und -1,4-diisocyanat, Bis(2-isocyanato-ethyl)fumarat, sowie Gemische aus zwei oder mehr davon, 1-Isocyanato-3,3,5-trimethyl-5-isocyanatomethylcyclohexan (Isophorondiisocyanat, IPDI), 2,4- und 2,6-Hexahydrotoluylendiisocyanat, Hexahydro-1,3- oder -1,4-phenylendiisocyanat, Benzidindiisocyanat, Naphthalin-1,5-diisocyanat, 1,6-Diisocyanato-2,2,4-trimethylhexan, 1,6-Diisocyanato-2,4,4-trimethylhexan, Xylylendi-isocyanat (XDI), Tetramethylxylylendiisocyanat (TMXDI), 1,3- und 1,4-Phenylendiisocyanat, 2,4- oder 2,6-Toluylendiisocyanat (TDI), 2,4'-Diphenylmethandiisocyanat, 2,2'-Diphenylmethandiisocyanat oder 4,4'-Diphenylmethandiisocyanat (MDI) oder deren partiell oder vollständig hydrierte Cycloalkylderivate, beispielsweise vollständig hydriertes MDI (H12-MDI), alkylsubstituierte Diphenylmethandiisocyanate, beispielsweise Mono-, Di-, Tri- oder Tetraalkyldiphenylmethandiisocyanat sowie deren partiell oder vollständig hydrierte Cycloalkylderivate, 4,4'-Diisocyanatophenylperfluorethan, Phthalsäure-bis-isocyanatoethylester, 1-Chlormethylphenyl-2,4- oder -2,6-diisocyanat, 1-Brommethylphenyl-2,4- oder -2,6-diisocyanat, 3,3-Bis-chlormethylether-4,4'-diphenyldiisocyanat, schwefelhaltige Diisocyanate, wie sie durch Umsetzung von 2 mol Diisocyanat mit 1 mol Thiodiglykol oder Dihydroxydihexylsulfid erhältlich sind, die Di- und Triisocyanate der Di- und Trimerfettsäuren, oder Gemische aus zwei oder mehr der genannten Diisocyanate, in Frage.

Ebenso kann man als Polyisocyanate sind drei- oder höherwertige Isocyanate, wie sie beispielsweise durch Oligomerisierung von Diisocyanaten, insbesondere durch Oligomerisierung der oben genannten Isocyanate, erhältlich sind, einsetzen. Beispiele für solche drei- und höherwertigen Polyisocyanate sind die Tri-isocyanurate von HDI oder IPDI oder deren Gemische oder deren gemischte Triisocyanurate sowie Polyphenylmethylenpolyisocyanat, wie es durch Phosgenierung von Anilin-Formaldehyd-Kondensationsprodukten erhältlich ist.

Zur Reduzierung der Viskosität der härtbaren Zusammensetzung lassen sich neben oder anstatt eines Reaktivverdünners auch Lösungsmittel und / oder Weichmacher einsetzen.

Als Lösungsmittel eignen sich aliphatische oder aromatische Kohlenwasserstoffe, halogenierte Kohlenwasserstoffe, Ketone, Ether, Ester, Esteralkohole, Ketoalkohole, Ketoether, Ketoester und Etherester.

Die hierin beschriebene Zusammensetzung kann ferner hydrophile Weichmacher enthalten. Diese dienen zur Verbesserung der Feuchtigkeitsaufnahme und damit zur Verbesserung der Reaktivität bei niedrigen Temperaturen. Als Weichmacher geeignet sind beispielsweise Ester der Abietinsäure, Adipinsäureester, Azelainsäureester, Benzoesäureester, Buttersäureester, Essigsäureester, Ester höherer Fettsäuren mit etwa 8 bis etwa 44 C-Atomen, epoxidierter Fettsäuren, Fettsäureester und Fette, Glykolsäureester, Phosphorsäureester, Phthalsäureester, von 1 bis 12 C-Atomen enthaltenden linearen oder verzweigten Alkoholen, Propionsäureester, Sebacinsäureester, Sulfonsäureester, Thiobuttersäureester, Trimellithsäureester, Zitronensäureester sowie Ester auf Nitrocellulose- und Polyvinylacetat-Basis, sowie Gemische aus zwei oder mehr davon.

Beispielsweise eignen sich von den Phthalsäureestern Dioctylphthalat, Dibutylphthalat, Diisoundecylphthalat oder Butylbenzylphthalat, von den Adipaten Dioctyladipat, Diisodecyladipat, Diisodecylsuccinat, Dibutylsebacat oder Butyloleat.

Ebenfalls als Weichmacher geeignet sind die reinen oder gemischten Ether monofunktioneller, linearer oder verzweigter C₄₋₁₆-Alkohole oder Gemische aus zwei oder mehr verschiedenen Ethern solcher Alkohole, beispielsweise Dioctylether (erhältlich als Cetiol OE, Fa. Cognis Deutschland GmbH, Düsseldorf).

Ferner eignen sich als Weichmacher endgruppenverschlossene Polyethylenglykole. Beispielsweise Polyethylen- oder Polypropylenglykoldi-C₁₋₄-alkylether, insbesondere die Dimethyl- oder Diethylether von Diethylenglykol oder Dipropylenglykol, sowie Gemische aus zwei oder mehr davon.

Besonders bevorzugt als Weichmacher sind jedoch endgruppenverschlossene Polyethylenglykole, wie Polyethylen- oder Polypropylenglykoldialkylether, wobei der Alkylrest ein bis vier C-Atome beträgt, und insbesondere die Dimethyl- und Diethylether von Diethylenglykol und Dipropylenglykol. Insbesondere mit Dimethyldiethylenglykol wird eine auch unter ungünstigeren Auftragungsbedingungen (geringe Luftfeuchtigkeit, niedrige Temperatur) eine akzeptable Aushärtung erreicht. Für weitere Einzelheiten zu Weichmachern wird auf die einschlägige Literatur der Technischen Chemie verwiesen.

Ebenfalls im Rahmen der vorliegenden Erfindung als Weichmacher geeignet sind Diurethane, welche sich beispielsweise durch Umsetzung von Diolen mit OH-Endgruppen mit monofunktionellen Isocyanaten herstellen lassen, indem die Stöchiometrie so gewählt wird, dass im Wesentlichen alle freien OH-Gruppen abreagieren. Gegebenenfalls überschüssiges Isocyanat kann anschließend beispielsweise durch Destillation aus dem Reaktionsgemisch entfernt werden. Eine weitere Methode zur Herstellung von Diurethanen besteht in der Umsetzung von monofunktionellen Alkoholen mit Diisocyanaten, wobei möglichst sämtliche NCO-Gruppen abreagieren.

Vorzugsweise weist die härtbare Zusammensetzung mindestens einen Weichmacher, insbesondere ein Polydimethylsiloxan auf.

Die härtbaren Zusammensetzungen enthalten den Weichmacher vorzugsweise in einer Menge von 1 bis 50 Gew.-%, bevorzugt in einer Menge von 10 bis 40 Gew.-%, besonders bevorzugt in einer Menge von 20 bis 30 Gew.-%, jeweils bezogen auf das Gesamtgewicht der Zusammensetzung. Wird ein Gemisch mehrerer Weichmacher eingesetzt, beziehen sich die Mengenangaben selbstverständlich auf die Gesamtmenge an Weichmacher in der Zusammensetzung.

Die hierin beschriebene Zusammensetzung kann außerdem bis zu etwa 20 Gew.-% an üblichen Haftvermittlern (Tackifier) enthalten. Als Haftvermittler geeignet sind beispielsweise Harze, Terpen-Oligomere, Cumaron-/lnden-Harze, aliphatische, petrochemische Harze und modifizierte Phenolharze. Geeignet sind im Rahmen der vorliegenden Erfindung beispielsweise Kohlenwasserstoffharze, wie sie durch Polymerisation von Terpenen, hauptsächlich *α*- oder *β*-Pinen, Dipenten oder Limonen gewonnen werden. Die Polymerisation dieser Monomere erfolgt in der Regel kationisch unter Initiierung mit Friedel-Crafts-Katalysatoren. Zu den Terpenharzen werden beispielsweise auch Copolymere aus Terpenen und anderen Monomeren, beispielsweise Styrol, *α*-Methylstyrol, Isopren und dergleichen, gerechnet. Die genannten Harze finden beispielsweise als Haftvermittler für Haftklebstoffe und Beschichtungsmaterialien Verwendung. Ebenfalls geeignet sind die Terpen-Phenol-Harze, die durch säurekatalysierte Addition von Phenolen an Terpene oder Kolophonium hergestellt werden. Terpen-Phenol-Harze sind in den meisten organischen Lösemitteln und Ölen löslich und mit anderen Harzen, Wachsen und Kautschuk mischbar. Ebenfalls im Rahmen der vorliegenden Erfindung als Zusatzstoff im oben genannten Sinne geeignet sind die Kolophoniumharze und deren Derivate, beispielsweise deren Ester.

Da insbesondere durch Gegenwart des Aminosilans die härtbaren Zusammensetzungen jedoch in der Regel bereits sehr gute Haftung zu sehr vielen Materialien zeigen, kann auf die Zugabe weiterer Haftvermittler oftmals verzichtet werden.

Vorzugsweise enthält die härtbare Zusammensetzung mindestens einen Stabilisator, ausgewählt aus Antioxidantien, UV-Stabilisatoren und Trockenmittel.

Als Antioxidantien kommen alle üblichen Antioxidantien in Frage. Sie sind vorzugsweise bis zu etwa 7 Gew.-%, insbesondere bis zu etwa 5 Gew.-% enthalten.

Die hierin Zusammensetzung kann UV-Stabilisatoren enthalten, die vorzugsweise bis zu etwa 2 Gew.-%, vorzugsweise etwa 1 Gew.-% eingesetzt werden. Als UV-Stabilisatoren besonders geeignet sind die sogenannten Hindered Amine Light Stabilisators (HALS). Es ist im Rahmen der vorliegenden Erfindung bevorzugt, wenn ein UV-Stabilisator eingesetzt wird, der eine Silylgruppe trägt und beim Vernetzen bzw. Aushärten in das Endprodukt eingebaut wird. Hierzu besonders geeignet sind die Produkte Lowilite 75, Lowilite 77 (Fa. Great Lakes, USA). Ferner können auch Benzotriazole, Benzophenone, Benzoate, Cyanacrylate, Acrylate, sterisch gehinderte Phenole, Phosphor und / oder Schwefel zugegeben werden.

Häufig ist es sinnvoll, die Zusammensetzungen durch Trockenmittel gegenüber eindringender Feuchtigkeit zu stabilisieren, um die Lagerbarkeit (shelf-life) noch weiter zu erhöhen.

Eine solche Verbesserung der Lagerbarkeit lässt sich beispielsweise durch den Einsatz von Trockenmitteln erreichen. Als Trockenmittel eignen sich alle Verbindungen, die mit Wasser unter Bildung einer gegenüber den in der Zubereitung vorliegenden reaktiven Gruppen inerten Gruppe reagieren, und hierbei möglichst geringe Veränderungen ihres Molekulargewichts eingehen. Weiterhin muss die Reaktivität der Trockenmittel gegenüber in die Zubereitung eingedrungener Feuchtigkeit höher sein, als die Reaktivität der Gruppen des in der Zubereitung vorliegenden erfindungsgemäßen silylgruppentragenden Polymers

Als Trockenmittel eignen sich beispielsweise Isocyanate.

Vorteilhafterweise werden als Trockenmittel aber Silane eingesetzt. Beispielsweise Vinylsilane wie 3-Vinylpropyltriethoxysilan, Oximsilane wie Methyl-O,O',O"-butan-2-on-trioximosilan oder O,O',O",O"'-Butan-2-ontetraoximosilan (CAS Nr. 022984-54-9 und 034206-40-1) oder Benzamidosilane wie Bis(N-methylbenzamido)methylethoxysilan (CAS Nr. 16230-35-6) oder Carbamatosilane wie Carbamatomethyltrimethoxysilan. Aber auch die Verwendung von Methyl-, Ethyl- oder Vinyltrimethoxysilan, Tetramethyl- oder -ethylethoxysilan ist möglich. Hinsichtlich Effizienz und Kosten sind hier Vinyltrimethoxysilan und Tetraethoxysilan besonders bevorzugt.

Ebenfalls als Trockenmittel geeignet sind die oben genannten Reaktivverdünner, sofern sie ein Molekulargewicht (Mₙ) von weniger als etwa 5.000 g/mol aufweisen und über Endgruppen verfügen, deren Reaktivität gegenüber eingedrungener Feuchtigkeit mindestens genauso groß, bevorzugt größer ist, als die Reaktivität der reaktiven Gruppen des erfindungsgemäß eingesetzten Polymers.

Schließlich können als Trockenmittel auch Alkylorthoformiate oder -orthoacetate eingesetzt werden, bsw. Methyl- oder Ethylorthoformiat, Methyl- oder Ethylorthoacetat,

Die Zusammensetzungen enthalten in der Regel etwa 0 bis etwa 6 Gew.-% Trockenmittel.

Die hierin beschriebene Zusammensetzung kann zusätzlich Füllstoffe enthalten. Hier eignen sich beispielsweise Kreide, Kalkmehl, gefällte und/oder pyrogene Kieselsäure, Zeolithe, Bentonite, Magnesiumcarbonat, Kieselgur, Tonerde, Ton, Talg, Titanoxid, Eisenoxid, Zinkoxid, Sand, Quartz, Flint, Glimmer, Glaspulver und andere gemahlene Mineralstoffe. Weiterhin können auch organische Füllstoffe eingesetzt werden, insbesondere Ruß, Graphit, Holzfasern, Holzmehl, Sägespäne, Zellstoff, Baumwolle, Pulpe, Baumwolle, Hackschnitzel, Häcksel und Spreu. Ferner können auch Kurzfasern wie Glasfaser, Glasfilament, Polyacrylnitril, Kohlefaser, Kevlarfaser oder auch Polyethylenfasern zugesetzt werden. Aluminiumpulver ist ebenfalls als Füllstoff geeignet.

Die pyrogenen und/oder gefällten Kieselsäuren weisen vorteilhaft eine BET-Oberfläche von 10 bis 90 m²/g auf. Bei Ihrer Verwendung bewirken sie keine zusätzliche Erhöhung der Viskosität der erfindungsgemäßen Zusammensetzung, tragen aber zu einer Verstärkung der gehärteten Zusammensetzung bei.

Es ist ebenso denkbar, pyrogene und/oder gefällte Kieselsäuren mit einer höheren BET-Oberfläche, vorteilhafterweise mit 100 bis 250 m²/g, insbesondere 110 bis 170 m²/g, als Füllstoff einzusetzen. Aufgrund der höheren BET-Oberfläche, kann man den gleichen Effekt, z.B. Verstärkung der gehärteten Zubereitung, bei einem geringeren Gewichtsanteil Kieselsäure erzielen. Somit kann man weitere Stoffe einsetzen, um die hierin beschriebene Zusammensetzung hinsichtlich anderer Anforderungen zu verbessern.

Ferner eignen sich als Füllstoffe Hohlkugeln mit einer mineralischen Hülle oder einer Kunststoffhülle. Dies können beispielsweise Glashohlkugeln sein, die unter den Handelsbezeichnungen Glass Bubbles® kommerziell erhältlich sind. Hohlkugeln auf Kunststoffbasis, z.B. Expancel® oder Dualite®, werden beispielsweise in der EP 0 520 426 B1 beschrieben. Diese sind aus anorganischen oder organischen Stoffen zusammengesetzt, jede mit einem Durchmesser von 1 mm oder weniger, bevorzugt von 500 µm oder weniger.

Für manche Anwendungen sind Füllstoffe bevorzugt, die den Zubereitungen Thixotropie verleihen. Solche Füllstoffe werden auch als rheologische Hilfsmittel beschrieben, z. B. hydrogenisiertes Rizinusöl, Fettsäureamide oder quellbare Kunststoffe wie PVC. Um gut aus einer geeigneten Dosiervorrichtung (z. B. Tube) auspressbar zu sein, besitzen solche Zubereitungen eine Viskosität von 3.000 bis 15.000, vorzugsweise 40.000 bis 80.000 mPas oder auch 50.000 bis 60.000 mPas.

Die Füllstoffe werden vorzugsweise in einer Menge von 1 bis 80 Gew.-%, besonders bevorzugt 2 bis 20 Gew.-%, und ganz besonders bevorzugt 5 bis 10 Gew.-%, jeweils bezogen auf das Gesamtgewicht der Zusammensetzung eingesetzt. Selbstverständlich können auch Gemische mehrerer Füllstoffe eingesetzt werden. In diesem Fall beziehen sich die Mengenangaben selbstverständlich auf die Gesamtmenge an Füllstoff in der Zusammensetzung.

Gegenstand der Erfindung ist weiterhin ein Verfahren zur Herstellung der erfindungsgemäßen Zusammensetzungen.

Die Herstellung der härtbaren Zusammensetzung kann durch einfaches Vermischen des Polyorganosiloxans, das mindestens eine, an ein Siliziumatom gebundene, Hydroxygruppe aufweist, des Silans der Formel (1), des Aminosilans, der Zinnverbindung und gegebenenfalls der weiteren Inhaltsstoffe erfolgen. Dies kann in geeigneten Dispergieraggregaten, z. B. einem Schnellmischer, geschehen. Vorzugsweise wird dabei darauf geachtet, dass die Mischung nach Möglichkeit nicht in Kontakt mit Feuchtigkeit kommt, der zu einem unerwünschten vorzeitigen Aushärten führen könnte. Entsprechende Maßnahmen sind hinlänglich bekannt und umfassen beispielsweise das Arbeiten in inerter Atmosphäre, etwa unter Schutzgas, und das Trocknen / Ausheizen einzelner Bestandteile, bevor diese zugemischt werden.

Ein bevorzugtes Herstellverfahren besteht darin, in einem ersten Schritt das Polyorganosiloxan, das mindestens eine, an ein Siliziumatom gebundene, Hydroxygruppe aufweist, und das Silan der Formel (1) zu vermischen, wobei dies in Gegenwart der gesamten Menge des Aminosilans oder eines Teils davon und gegebenenfalls mindestens eines Weichmachers erfolgt, in einem zweiten Schritt den verbleibenden Teil des Aminosilans und gegebenenfalls weitere Inhaltsstoffe zuzugeben und alle Bestandteile zu vermischen, und in einem dritten Schritt die Zinnverbindung zuzugeben und mit den übrigen Bestandteilen zu vermischen.

Gegenstand der Erfindung ist zudem die Verwendung der erfindungsgemäßen Zusammensetzungen als Kleb-, Dicht- oder Beschichtungsstoff.

Die Zusammensetzung kann beispielsweise als Klebstoff, Dichtmasse, Spachtelmasse und zur Herstellung von Formteilen verwendet werden. Ein weiteres Anwendungsgebiet der Zusammensetzungen ist die Verwendung als Dübel-, Loch- oder Rissspachtelmasse. Bevorzugt ist der Einsatz als Dichtstoff.

Die Zusammensetzungen eignen sich unter anderem zum Verkleben von Kunststoffen, Metallen, Glas, Keramik, Holz, Holzwerkstoffen, Papier, Papierwerkstoffen, Gummi und Textilien, zum Verkleben von Fußböden, Abdichten von Bauwerksteilen, Fenstern, Wand- und Bodenbelägen sowie Fugen im allgemeinen. Hierbei können die Materialien jeweils mit sich selbst oder beliebig untereinander verklebt werden.

Die folgenden Beispiele dienen der Erläuterung der Erfindung, die Erfindung ist aber nicht darauf beschränkt.

### Beispiele

### Beispiel 1:

Durch Mischen der in Tabelle 1 aufgeführten Rohstoffe wurden die Vergleichszusammensetzungen VB1 und VB3, sowie die erfindungsgemäße Zusammensetzung B2 hergestellt. Alle drei Formulierungen basieren auf den gleichen Rohstoffen, unterscheiden sich aber im Mengenverhältnis von Amniosilan zu Zinnverbindung. Nur Zusammensetzung B2 enthält diese Bestandteile im erfindungsgemäß geforderten Verhältnis.

**Tabelle 1**

| | **VB1** | **B2** | **VB3** |
|---|---|---|---|
| **Rohstoffe** | **Gew.-Teile** | **Gew.-Teile** | **Gew.-Teile** |
| α,ω-dihydroxy-terminiertes Polydimethylsiloxan mit Viskosität von 80.000 cST | 59,6 | 59,6 | 60,3 |
| Polydimethylsiloxan mit Viskosität von 1000 cST | 26,6 | 26,6 | 26,9 |
| Vinyl-tris(ethyllactato)silan | 5,0 | 5,0 | 5,0 |
| Hochdisperse Kieselsäure | 7,4 | 7,4 | 7,4 |
| Aminosilan (Gemisch aus 3-Aminopropyltrimethoxysilan und 3-(N,N-Dimethylamino)-propyltrimethoxysilan im Gewichtsverhältnis1,4:1) | 1,2 | 1,2 | 0,002 |
| Zinnverbindung (Di-n-butylzinndilaurat (DBTL)) | 0,02 | 0,2 | 0,2 |
| | | | |
| Molverhältnis Zinnverbindung zu Aminosilan | 1 : 234 | 1 : 23 | 1 : 0,02 |

Die hergestellten Formulierungen wurden hinsichtlich Hautbildungszeit, Härte, Dehnbarkeit und Dehnung untersucht. Alle Tests wurden für die frisch formulierte Zusammensetzung und nach 4 und 12 Wochen Alterung bei 40°C/80% relative Luftfeuchtigkeit durchgeführt. Die Ergebnisse für die Vergleichsformulierung VB1 sind in Tabelle 2 dargestellt, die Ergebnisse für die erfindungsgemäße Formulierung B2 in Tabelle 3. Die Vergleichsformulierung VB3 vernetzt bereits beim Vermischen der Rohstoffe, so dass keine mechanischen Eigenschaften bestimmt werden konnten.

Ein Vergleich der Ergebnisse für die Vergleichsformulierung VB1 und die erfindungsgemäße Formulierung B2 zeigt, dass sich für die Vergleichsformulierung nach Lagerung kein praktikables Härtungsverhalten erzielen lässt, und die mechanischen Eigenschaften des ausgehärteten Produkts unzureichend sind. Bereits nach 12 Wochen Alterung unter den angegebenen Bedingungen lässt sich die Formulierung überhaupt nicht mehr aushärten. Die Formulierung weist demnach keine hinreichende Lagerstabilität auf.

**Tabelle 2: Eigenschaften der Vergleichsformulierung VB1**

| **Formulierung VB1** | **frisch** | **4 Wochen Lagerung bei 40% relative Luftfeuchtigkeit und 80 °C** | **12 Wochen Lagerung bei 40% relative Luftfeuchtigkeit und 80 °C** |
|---|---|---|---|
| Hautbildungszeit (min) | 12 | 38 | Nicht messbar |
| Shore A 1d | Nicht messbar | Nicht messbar | Nicht messbar |
| Shore A 7d | 11 | Nicht messbar | Nicht messbar |
| Härtungstiefe (mm / 24 h) | 3,1 | Nicht messbar | Nicht messbar |
| E-Modul bei 100 % (N/mm²) | Nicht messbar | Nicht messbar | Nicht messbar |
| Bruchkraft (N/mm²) | Nicht messbar | Nicht messbar | Nicht messbar |
| Bruchdehnung (%) | Nicht messbar | Nicht messbar | Nicht messbar |

**Tabelle 3: Eigenschaften der erfindungsgemäßen Formulierung B2**

| **Formulierung B2** | **frisch** | **4 Wochen Lagerung bei 40% relative Luftfeuchtigkeit und 80 °C** | **12 Wochen Lagerung bei 40% relative Luftfeuchtigkeit und 80 °C** |
|---|---|---|---|
| Hautbildungszeit (min) | 18 | 27 | 45 |
| Shore A 1d | 22 | 16 | 11 |
| Shore A 7d | 30 | 29 | 25 |
| Härtungstiefe (mm / 24 h) | 3,6 | 3,8 | 3,7 |
| E-Modul bei 100 % (N/mm²) | 0,34 | 0,31 | 0,26 |
| Bruchkraft (N/mm²) | 1,05 | 1,02 | 1,02 |
| Bruchdehnung (%) | 450 | 452 | 489 |

### Messung der Hautbildungszeit:

Die Bestimmung der Hautbildungszeit erfolgt im Normalklima (23 +/- 2°C, relative Luftfeuchte 50 +/- 5 %). Die Temperatur des Dichtstoffes muss 23 +/-2°C betragen, der Dichtstoff ist mindestens 24h im Labor vorzulagern. Der Dichtstoff wird auf ein Blatt Papier aufgebracht und mit einem Ziehspachtel zu einem Fell ausgezogen (Dicke ca. 2 mm, Breite ca. 7 cm). Stoppuhr sofort starten. In Intervallen die Oberfläche leicht mit der Fingerspitze berühren und Finger wieder wegziehen - so stark auf die Oberfläche drücken, dass ein Abdruck auf der Oberfläche bei Erreichen der Hautbildungzeit verbleibt. Die Hautbildungszeit ist erreicht, wenn keine Dichtmasse mehr an der Fingerspitze haften bleibt. Die Hautbildungszeit wird in Minuten angegeben.

### Messung der Shore A Härte:

Die Durchführung erfolgt gemäß an ISO 868.

### Messung der Härtungstiefe:

Ein Dichtstoffstrang mit einer Höhe von 10 mm (+/- 1 mm) und einer Breite von 20 mm (+/- 2 mm) wird mit einem entsprechenden Spachtel auf ein Kunststoffkartenblatt appliziert. Nach einer Lagerung von 24 Stunden bei Normalklima (23 +/- 2 °C, relative Luftfeuchte 50 +/- 5 %), wird ein Stück aus dem Strang herausgeschnitten und die Dicke der ausgehärteten Schicht mit einer Schieblehre gemessen. Die Härtungstiefe wird in [mm / 24 h] angegeben.

### Messung der mechanischen Eigenschaften (Zugversuch):

Mit dem Zugversuch werden die Bruchkraft, Bruchdehnung und Dehnspannungswerte (E-Module) in Anlehnung an DIN 53504 ermittelt.

Abweichung zur Norm: Als Probekörper werden Schulterstäbe mit folgenden Dimensionen verwendet: Dicke: 2 +/- 0.2 mm; Breite des Stegs: 10 +/- 0,5 mm; Länge des Stegs: ca. 45 mm; Gesamtlänge: 9 cm. Die Prüfung erfolgt bei im Normklima (23 +/- 2 °C, 50 +/- 5 % rel. Luftfeuchtigkeit). Die Prüfung erfolgt nach 7 Tagen Aushärtung.

Durchführung: Von der Masse wird ein 2 mm dicker Film ausgezogen. Der Film wird für 7 Tage im Normklima gelagert und dann die Schulterstäbe herausgestanzt. Für jede Bestimmung sind je drei Schulterstäbe herzustellen. Die Prüfung ist im Normklima durchzuführen. Die Prüflinge müssen vor der Messung mindestens 20 Minuten an die Prüftemperatur angeglichen (d.h. gelagert) werden. Vor der Messung ist die Dicke der Probekörper an mindestens 3 Stellen bei Raumtemperatur mit einer Schieblehre zu messen, d.h. bei den Schulterstäben sind innerhalb der Anfangsmesslänge vorzugsweise die Enden und die Mitte zu vermessen. Bei elastischen Materialien empfiehlt es sich zusätzlich noch quer über den Steg zu messen. Der Mittelwert ist in das Messprogramm einzugeben. Die Probekörper sind so in die Zugprüfmaschine einzuspannen, dass die Längsachse mit der mechanischen Achse der Zugprüfmaschine übereinstimmt und eine möglichst große Fläche der Stabköpfe erfasst wird, ohne dass der Steg eingeklemmt wird. Mit einer Vorschubgeschwindigkeit von 50 mm/min wir der Schulterstab auf eine Vorspannung von <0,1 MPa gespannt. Dann erfolgt die Aufnahme der Kraft-Längenänderungskurve mit einer Vorschubgeschwindigkeit von 50 mm/min.

Auswertung: Folgende Werte sind der Messung zu entnehmen: Bruchkraft in [N/mm²], Bruchdehnung in [%] und E-Modul bei 100% Dehnung in [N/mm²].

## Patentansprüche

1. Härtbare Zusammensetzung enthaltend
(A) mindestens ein Polyorganosiloxan, das mindestens eine, an ein Siliziumatom gebundene, Hydroxygruppe aufweist,
(B) mindestens ein Silan der Formel (1):
Si(R¹)ₘ(R²)ₙ(R³)₄₋₍ₘ₊ₙ₎ (1)
wobei
jedes R¹ unabhängig steht für:
einen substituierten oder unsubstituierten Alkyl-, Alkenyl- oder Alkinylrest;
einen substituierten oder unsubstituierten cycloaliphatischen Rest oder Arylrest;
einen substituierten oder unsubstituierten heteroalicyclischen Rest oder Heteroarylrest;
jedes R² unabhängig steht für einen Rest der allgemeinen Formel (2):
-OCR⁴₂COOR⁵ (2)
wobei
jedes R⁴ unabhängig steht für:
Wasserstoff; oder
einen substituierten oder unsubstituierten Alkyl-, Alkenyl- oder Alkinylrest;
R⁵ steht für:
einen substituierten oder unsubstituierten Alkyl-, Alkenyl- oder Alkinylrest;
jedes R³ unabhängig steht für einen Rest der allgemeinen Formel (3):
-OCR⁶₂CONR⁷R⁸ (3)
wobei
jedes R⁶ unabhängig steht für:
Wasserstoff oder
einen substituierten oder unsubstituierten Alkyl-, Alkenyl- oder Alkinylrest;
R⁷ steht für:
Wasserstoff,
einen substituierten oder unsubstituierten Alkyl-, Alkenyl- oder Alkinylrest,
einen substituierten oder unsubstituierten cycloaliphatischen Rest oder Arylrest, R⁸, oder
einen Rest -(CH₂)_{q}-COOR⁹, wobei q eine ganze Zahl von 2 bis 10, insbesondere 2 ist und R⁹ für einen substituierten oder unsubstituierten Alkyl-, Alkenyl- oder Alkinylrest,
oder einen substituierten oder unsubstituierten cycloaliphatischen Rest oder Arylrest steht;
R⁸ steht für einen Rest der allgemeinen Formel (4):
-R¹⁰-SiR¹¹ₒ(OR¹²)₃₋ₒ (4)
wobei
R¹⁰ steht für:
einen, gegebenenfalls durch ein Heteroatom unterbrochenen Alkylenrest;
jedes R¹¹ unabhängig steht für:
einen substituierten oder unsubstituierten Alkyl-, Alkenyl- oder Alkinylrest;
jedes R¹² unabhängig steht für:
einen substituierten oder unsubstituierten Alkyl-, Alkenyl- oder Alkinylrest,
einen Acylrest,
oder einen Rest der Formel (5):
-CR¹³₂COOR¹⁴ (5)
wobei
jedes R¹³ unabhängig steht für:
Wasserstoff; oder
einen substituierten oder unsubstituierten Alkyl-, Alkenyl- oder Alkinylrest;
R¹⁴ steht für:
einen substituierten oder unsubstituierten Alkyl-, Alkenyl- oder Alkinylrest; und
o unabhängig für 0, 1 oder 2 steht, und
m unabhängig für 0 oder 1 und n unabhängig für 0, 1, 2, 3 oder 4 steht, wobei die Summe n + m maximal 4 beträgt,
(C) mindestens ein Aminosilan, und
(D) mindestens eine Zinnverbindung,
**dadurch gekennzeichnet, dass** das Molverhältnis von Aminosilan zu Zinnverbindung 1 : 1 bis 50 : 1 beträgt.

2. Härtbare Zusammensetzung gemäß Anspruch 1, **dadurch gekennzeichnet, dass** das Molverhältnis von Aminosilan zu Zinnverbindung 10 : 1 bis 40 : 1, vorzugsweise 20 : 1 bis 35 : 1 beträgt.

3. Härtbare Zusammensetzung gemäß mindestens einem der Ansprüche 1 bis 2, **dadurch gekennzeichnet, dass** das Polyorganosiloxan, das mindestens eine, an ein Siliziumatom gebundene, Hydroxygruppe aufweist, ein Polydiorganosiloxan, vorzugsweise ein Polydimethylsiloxan, ist, das mindestens eine, vorzugsweise mindestens zwei terminale Hydroxygruppen aufweist.

4. Härtbare Zusammensetzung gemäß mindestens einem der Ansprüche 1 bis 3, **dadurch gekennzeichnet, dass** das Polyorganosiloxan, das mindestens eine, an ein Siliziumatom gebundene, Hydroxygruppe aufweist, ein α,ω-dihydroxyterminiertes Polydiorganosiloxan, insbesondere ein α,ω-dihydroxyterminiertes Polydimethylsiloxan ist.

5. Härtbare Zusammensetzung gemäß mindestens einem der Ansprüche 1 bis 4, **dadurch gekennzeichnet, dass** das Silan ein Silan der Formel (1) ist, wobei
jedes R¹ unabhängig voneinander für einen Alkylrest mit 1 bis 10 Kohlenstoffatomen, insbesondere Methyl, Ethyl, Propyl oder Isopropyl, für einen Alkenylrest mit 2 bis 10 Kohlenstoffatomen, insbesondere Vinyl oder Allyl, oder einen Arylrest mit 6 bis 10 Kohlenstoffatomen, insbesondere Phenyl, steht, und/oder
jedes R² unabhängig voneinander für einen Rest der Formel (2) steht, wobei einer der Reste R⁴ für Wasserstoff und der zweite der Reste R⁴ für Wasserstoff oder einen substituierten oder unsubstituierten Alkylrest mit 1 bis 10 Kohlenstoffatomen, insbesondere Methyl, und R⁵ für einen substituierten oder unsubstituierten Alkylrest mit 1 bis 10 Kohlenstoffatomen, insbesondere mit 1 bis 4 Kohlenstoffatomen, besonders bevorzugt Methyl oder Ethyl, steht.

6. Härtbare Zusammensetzung gemäß mindestens einem der Ansprüche 1 bis 5, **dadurch gekennzeichnet, dass** das Silan ein Silan der Formel (1) ist, wobei die Summe n + m 4 beträgt.

7. Härtbare Zusammensetzung gemäß mindestens einem der Ansprüche 1 bis 6, **dadurch gekennzeichnet, dass** das Silan der Formel (1) ausgewählt ist aus Methyltris(ethyllactato)silan, Ethyl-tris(ethyllactato)silan, Phenyl-tris(ethyllactato)silan, Vinyl-tris(ethyllactato)silan, Tetra(ethyllactato)silan und Mischungen davon.

8. Härtbare Zusammensetzung gemäß mindestens einem der Ansprüche 1 bis 5, **dadurch gekennzeichnet, dass** das Silan ein Silan der Formel (1) ist, wobei
die Summe n + m maximal 3 und
jedes R³ unabhängig voneinander für einen Rest der Formel (3) steht, wobei einer der Reste R⁶ für Wasserstoff und der zweite der Reste R⁶ für Wasserstoff oder einen substituierten oder unsubstituierten Alkylrest mit 1 bis 10 Kohlenstoffatomen, insbesondere Methyl, R⁷ für Wasserstoff, einen substituierten oder unsubstituierten Alkylrest mit 1 bis 10 Kohlenstoffatomen, insbesondere mit 1 bis 4 Kohlenstoffatomen, und R⁸ für einen Rest der Formel (4) steht, wobei R¹⁰ ein Alkylenrest der Formel -(CH₂)ₚ- ist, wobei p eine ganze Zahl von 1 bis 6, insbesondere 3 ist, jedes R¹¹ unabhängig voneinander für einen substituierten oder unsubstituierten Alkylrest mit 1 bis 10 Kohlenstoffatomen, insbesondere mit 1 bis 4 Kohlenstoffatomen, besonders bevorzugt Methyl oder Ethyl, und jedes R¹² unabhängig voneinander für einen substituierten oder unsubstituierten Alkylrest mit 1 bis 10 Kohlenstoffatomen, insbesondere mit 1 bis 4 Kohlenstoffatomen, besonders bevorzugt Methyl oder Ethyl, steht.

9. Härtbare Zusammensetzung gemäß mindestens einem der Ansprüche 1 bis 8, **dadurch gekennzeichnet, dass** das Aminosilan ein Aminosilan der Formel (6) ist,
(R¹⁵R⁷N)-R¹⁰-SiR¹¹ₒ(OR¹²)₃₋ₒ (6)
wobei
o, R⁷, R¹⁰, sowie jedes R¹¹ und jedes R¹², jeweils unabhängig voneinander, die in Anspruch 1 angegebenen Bedeutungen haben, und
R¹⁵ steht für:
Wasserstoff,
einen substituierten oder unsubstituierten Alkyl-, Alkenyl- oder Alkinylrest.

10. Härtbare Zusammensetzung gemäß mindestens einem der Ansprüche 1 bis 9, **dadurch gekennzeichnet, dass** das Aminosilan ausgewählt ist aus 3-Aminopropyltrimethoxysilan, 3-Aminopropyltriethoxysilan, Aminomethyltrimethoxysilan, Aminomethyltriethoxysilan, 3-(N,N-Dimethylamino)propyltrimethoxysilan, 3-(N,N-Dimethylamino)propyltriethoxysilan, (N,N-Dimethylamino)methyltrimethoxysilan, (N,N-Dimethylamino)methyltriethoxysilan, Bis(trimethoxysilylpropyl)amin, Bis(3-triethoxysilyl)propylamin und Mischungen davon.

11. Härtbare Zusammensetzung gemäß mindestens einem der Ansprüche 1 bis 10, **dadurch gekennzeichnet, dass** die Zinnverbindung eine zinnorganische Verbindung ist, ausgewählt aus 1,3-Dicarbonylverbindungen des zwei- bzw. vierwertigen Zinns, den Dialkylzinn-(IV)-Dicarboxylaten, den Dialkylzinn-(IV)-Dialkoxylaten, den Dialkylzinn-(IV)-oxiden, den Zinn-(II)-carboxylaten und Mischungen davon.

12. Verfahren zur Herstellung der härtbaren Zusammensetzung nach mindestens einem der Ansprüche 1 bis 11, **dadurch gekennzeichnet, dass** das Polyorganosiloxan, das mindestens eine, an ein Siliziumatom gebundene, Hydroxygruppe aufweist, das Silan der Formel (1), das Aminosilan, die Zinnverbindung und gegebenenfalls mindestens ein weiterer Inhaltsstoff miteinander gemischt werden.

13. Verfahren gemäß Anspruch 12, **dadurch gekennzeichnet, dass** in einem ersten Schritt das Polyorganosiloxan, das mindestens eine, an ein Siliziumatom gebundene, Hydroxygruppe aufweist, und das Silan der Formel (1) vermischt werden, wobei dies in Gegenwart der gesamten Menge des Aminosilans oder eines Teils davon und gegebenenfalls mindestens eines Weichmachers erfolgt, in einem zweiten Schritt der verbleibende Teil des Aminosilans und gegebenenfalls weitere Inhaltsstoffe zugegeben und alle Bestandteile vermischt werden, und in einem dritten Schritt die Zinnverbindung zugegeben und mit den übrigen Bestandteilen vermischt wird.

14. Verwendung einer härtbaren Zusammensetzung gemäß mindestens einem der Ansprüche 1 bis 11 oder einer härtbaren Zusammensetzung hergestellt nach einem Verfahren gemäß mindestens einem der Ansprüche 12 bis 13 als Kleb-, Dicht- oder Beschichtungsstoff.

## Claims

1. A curable composition containing
(A) at least one polyorganosiloxane comprising at least one hydroxy group bonded to a silicon atom,
(B) at least one silane of formula (1):
Si(R¹)ₘ(R²)ₙ(R³)₄₋₍ₘ₊ₙ₎ (1)
where
each R¹ independently represents:
a substituted or unsubstituted alkyl, alkenyl or alkinyl residue;
a substituted or unsubstituted cycloaliphatic residue or aryl residue;
a substituted or unsubstituted heteroalicyclic residue or heteroaryl residue;
each R² independently represents a residue of general formula (2):
-OCR⁴₂COOR⁵ (2)
where
each R⁴ independently represents:
hydrogen; or
a substituted or unsubstituted alkyl, alkenyl or alkinyl residue;
R⁵ represents:
a substituted or unsubstituted alkyl, alkenyl or alkinyl residue;
each R³ independently represents a residue of general formula (3):
-OCR⁶₂CONR⁷R⁸ (3)
where
each R⁶ independently represents:
hydrogen or
a substituted or unsubstituted alkyl, alkenyl or alkinyl residue;
R⁷ represents:
hydrogen,
a substituted or unsubstituted alkyl, alkenyl or alkinyl residue,
a substituted or unsubstituted cycloaliphatic residue or aryl residue,
R⁸, or
a residue -(CH₂)_{q}-COOR⁹, where q is an integer from 2 to 10, in particular 2, and R⁹ represents a substituted or unsubstituted alkyl, alkenyl or alkinyl residue, or a substituted or unsubstituted cycloaliphatic residue or aryl residue;
R⁸ represents a residue of general formula (4):
-R¹⁰-SiR¹¹ₒ(OR¹²)₃₋ₒ (4)
where
R10 represents:
an alkylene residue which is optionally interrupted by a heteroatom;
each R¹¹ independently represents:
a substituted or unsubstituted alkyl, alkenyl or alkinyl residue;
each R¹² independently represents:
a substituted or unsubstituted alkyl, alkenyl or alkinyl residue,
an acyl residue,
or a residue of formula (5):
-CR¹³₂COOR¹⁴ (5)
where
each R¹³ independently represents:
hydrogen; or
a substituted or unsubstituted alkyl, alkenyl or alkinyl residue;
R¹⁴ represents:
a substituted or unsubstituted alkyl, alkenyl or alkinyl residue; and
o independently represents 0, 1 or 2, and
m independently represents 0 or 1, and n independently represents 0, 1, 2, 3 or 4, where the sum n + m equals at most 4,
(C) at least one aminosilane, and
(D) at least one tin compound,
**characterized in that** the molar ratio of aminosilane to tin compound is from 1:1 to 50:1.

2. The curable composition according to claim 1, **characterized in that** the molar ratio of aminosilane to tin compound is from 10:1 to 40:1, preferably from 20:1 to 35:1.

3. The curable composition according to at least one of claims 1 to 2, **characterized in that** the polyorganosiloxane comprising at least one hydroxy group bonded to a silicon atom is a polydiorganosiloxane, preferably a polydimethylsiloxane, comprising at least one, preferably at least two, terminal hydroxy groups.

4. The curable composition according to at least one of claims 1 to 3, **characterized in that** the polyorganosiloxane comprising at least one hydroxy group bonded to a silicon atom is a α,ω-dihydroxy-terminated polydiorganosiloxane, in particular a α,ω-dihydroxy-terminated polydimethylsiloxane.

5. The curable composition according to at least one of claims 1 to 4, **characterized in that** the silane is a silane of formula (1), where
each R¹ independently represents an alkyl residue having 1 to 10 carbon atoms, in particular methyl, ethyl, propyl or isopropyl, an alkenyl residue having 2 to 10 carbon atoms, in particular vinyl or allyl, or an aryl residue having 6 to 10 carbon atoms, in particular phenyl, and/or
each R² independently represents a residue of formula (2), where one of the residues R⁴ represents hydrogen and the second of the residues R⁴ represents hydrogen or a substituted or unsubstituted alkyl residue having 1 to 10 carbon atoms, in particular methyl, and R⁵ represents a substituted or unsubstituted alkyl residue having 1 to 10 carbon atoms, in particular having 1 to 4 carbon atoms, particularly preferably methyl or ethyl.

6. The curable composition according to at least one of claims 1 to 5, **characterized in that** the silane is a silane of formula (1), where the sum n + m equals 4.

7. The curable composition according to at least one of claims 1 to 6, **characterized in that** the silane of formula (1) is selected from methyl-tris(ethyllactato)silane, ethyl-tris(ethyllactato)silane, phenyl-tris(ethyllactato)silane, vinyl-tris(ethyllactato)silane, tetra(ethyllactato)silane and mixtures thereof.

8. The curable composition according to at least one of claims 1 to 5, **characterized in that** the silane is a silane of formula (1), where
the sum n + m is at most 3, and
each R³ represents, independently of one another, a residue of formula (3), where one of the residues R⁶ represents hydrogen and the second of the residues R⁶ represents hydrogen or a substituted or unsubstituted alkyl residue having 1 to 10 carbon atoms, in particular methyl, R⁷ represents hydrogen, a substituted or unsubstituted alkyl residue having 1 to 10 carbon atoms, in particular having 1 to 4 carbon atoms, and R⁸ represents a residue of formula (4), where R¹⁰ is an alkylene residue of formula -(CH₂)ₚ-, where p is an integer from 1 to 6, in particular 3, each R¹¹ represents, independently of one another, a substituted or unsubstituted alkyl residue having 1 to 10 carbon atoms, in particular having 1 to 4 carbon atoms, particularly preferably methyl or ethyl, and each R¹² represents, independently of one another, a substituted or unsubstituted alkyl residue having 1 to 10 carbon atoms, in particular having 1 to 4 carbon atoms, particularly preferably methyl or ethyl.

9. The curable composition according to at least one of claims 1 to 8, **characterized in that** the aminosilane is an aminosilane of formula (6),
(R¹⁵R⁷N)-R¹⁰-SiR¹¹ₒ(OR¹²)₃₋ₒ (6)
where
o, R⁷, R¹⁰, each R¹¹ and each R¹² each have, independently of one another, the meanings specified in claim 1, and
R¹⁵ represents:
hydrogen,
a substituted or unsubstituted alkyl, alkenyl or alkinyl residue.

10. The curable composition according to at least one of claims 1 to 9, **characterized in that** the aminosilane is selected from 3-aminopropyltrimethoxysilane, 3-aminopropyltriethoxysilane, aminomethyltrimethoxysilane, aminomethyltriethoxysilane, 3-(N,N-dimethylamino)propyltrimethoxysilane, 3-(N,N-dimethylamino)propyltriethoxysilane, (N,N-dimethylamino)methyltrimethoxysilane, (N,N-dimethylamino)methyltriethoxysilane, bis(trimethoxysilylpropyl)amine, bis(3-triethoxysilyl)propylamine and mixtures thereof.

11. The curable composition according to at least one of claims 1 to 10, **characterized in that** the tin compound is an organotin compound selected from 1,3-dicarbonyl compounds of divalent or tetravalent tin, dialkyltin (IV) dicarboxylates, dialkyltin (IV) dialkoxylates, dialkyltin (IV) oxides, tin (II) carboxylates, and mixtures thereof.

12. A method for preparing the curable composition according to at least one of claims 1 to 11, **characterized in that** the polyorganosiloxane comprising at least one hydroxy group bonded to a silicon atom, the silane of formula (1), the aminosilane, the tin compound and optionally at least one further ingredient are mixed together.

13. The method according to claim 12, **characterized in that** the polyorganosiloxane comprising at least one hydroxy group bonded to a silicon atom and the silane of formula (1) are mixed in a first step, this being carried out in the presence of the entire amount of the aminosilane or a partial amount thereof and optionally at least one plasticizer, the remaining amount of the aminosilane and optionally further ingredients being added and all components being mixed in a second step, and the tin compound being added and mixed with the other components in a third step.

14. The use of a curable composition according to at least one of claims 1 to 11 or of a curable composition prepared using a method according to at least one of claims 12 to 13 as an adhesive, sealant or coating substance.

## Revendications

1. Composition durcissable contenant
(A) au moins un polyorganosiloxane comportant au moins un groupe hydroxyle attaché à un atome de silicium,
(B) au moins un silane de la formule (1) :
Si(R¹)ₘ(R²)ₙ(R³)₄₋₍ₘ₊ₙ₎ (1)
dans laquelle
chaque R¹ représente indépendamment :
un radical alkyle, alcényle ou alcynyle substitué ou non substitué ;
un radical cycloaliphatique ou aryle substitué ou non substitué ;
un radical hétéroalicyclique ou hétéroaryle substitué ou non substitué ;
chaque R² représente indépendamment un radical de la formule générale (2) :
-OCR⁴₂COOR⁵ (2)
dans laquelle
chaque R⁴ représente indépendamment :
un atome d'hydrogène ; ou
un radical alkyle, alcényle ou alcynyle substitué ou non substitué ;
R⁵ représente :
un radical alkyle, alcényle ou alcynyle substitué ou non substitué ;
chaque R³ représente indépendamment un radical de la formule générale (3) :
-OCR⁶₂CONR⁷R⁸ (3)
dans laquelle
chaque R⁶ représente indépendamment :
un atome d'hydrogène ou
un radical alkyle, alcényle ou alcynyle substitué ou non substitué ;
R⁷ représente :
un atome d'hydrogène,
un groupe alkyle, alcényle ou alcynyle, substitué ou non substitué,
un radical cycloaliphatique ou un radical aryle substitué ou non substitué,
R⁸ ou
un radical -(CH₂)_{q}-COOR⁹, q étant un nombre entier de 2 à 10, en particulier 2,
et R⁹ représente un radical alkyle, alcényle ou alcynyle substitué ou non substitué, ou un radical aryle ou cycloaliphatique substitué ou non substitué ;
R⁸ représente un radical de la formule générale (4) :
-R¹⁰-SiR¹¹ₒ(OR¹²)₃₋ₒ (4)
dans laquelle
R¹⁰ représente :
un radical alkylène éventuellement interrompu par hétéroatome ;
chaque R¹¹ représente indépendamment :
un radical alkyle, alcényle ou alcynyle substitué ou non substitué ;
chaque R¹² représente indépendamment :
un radical alkyle, alcényle ou alcynyle substitué ou non substitué,
un radical acyle,
ou un radical de la formule (5) :
-CR¹³₂COOR¹⁴ (5)
dans laquelle
chaque R¹³ représente indépendamment :
un atome d'hydrogène ; ou
un radical alkyle, alcényle ou alcynyle substitué ou non substitué ;
R¹⁴ représente :
un radical alkyle, alcényle ou alcynyle substitué ou non substitué ; et
o est indépendamment 0, 1 ou 2, et
m représente indépendamment 0 ou 1 et n représente indépendamment 0, 1, 2, 3 ou 4, la somme n + m étant égale à 4 maximum,
(C) au moins un aminosilane, et
(D) au moins un composé de l'étain,
**caractérisée en ce que** le rapport molaire de l'aminosilane au composé de l'étain est de 1:1 à 50:1.

2. Composition durcissable selon la revendication 1, **caractérisée en ce que** le rapport molaire de l'aminosilane au composé de l'étain est de 10:1 à 40:1, de préférence 20:1 à 35:1.

3. Composition durcissable selon l'une des revendications 1 à 2, **caractérisée en ce que** le polyorganosiloxane, comportant au moins un groupe hydroxyle lié à un atome de silicium, comporte un polydiorganosiloxane, de préférence un polydiméthylsiloxane comportant au moins un, de préférence au moins deux, groupes hydroxyle terminaux.

4. Composition durcissable selon l'une des revendications 1 à 3, **caractérisée en ce que** le polyorganosiloxane, comportant au moins un groupe hydroxyle lié à un atome de silicium, est un polydiorganosiloxane à terminaison α,ω-dihydroxyle, en particulier un polydiméthylsiloxane à terminaison α,ω-dihydroxyle.

5. Composition durcissable selon l'une des revendications 1 à 4, **caractérisée en ce que** le silane est un silane de la formule (1),
chaque R¹ représentant indépendamment un radical alkyle ayant 1 à 10 atomes de carbone, en particulier un méthyle, éthyle, propyle ou isopropyle, un radical alcényle ayant 2 à 10 atomes de carbone, notamment un vinyle ou allyle, ou un radical aryle ayant 6 à 10 atomes de carbone, en particulier un phényle, et/ou
chaque R² représentant indépendamment un radical de la formule (2), un premier des radicaux R⁴ étant un atome d'hydrogène et le deuxième des radicaux R⁴ étant un atome d'hydrogène ou un radical alkyle substitué ou non substitué ayant 1 à 10 atomes de carbone, en particulier un méthyle, et R⁵ représentant un radical alkyle substitué ou non substitué ayant 1 à 10 atomes de carbone, en particulier ayant 1 à 4 atomes de carbone, plus préférablement un méthyle ou éthyle.

6. Composition durcissable selon l'une des revendications 1 à 5, **caractérisée en ce que** le silane est un silane de la formule (1), dont la somme n + m est égale à 4.

7. Composition durcissable selon l'une des revendications 1 à 6, **caractérisée en ce que** le silane de la formule (1) est choisi parmi le méthyl-tris(éthyllactato)silane, l'éthyl-tris (éthyllactato)silane, le phényl-tris(éthyllactato)silane, le vinyl-tris(éthyllacto)silane, le tétra(éthyllacto)silane et leurs mélanges.

8. Composition durcissable selon l'une des revendications 1 à 5, **caractérisée en ce que** le silane est un silane de la formule (1), dans laquelle
la somme n + m est égale à 3 maximum, et
chaque R³ représente indépendamment un radical de la formule (3), l'un des radicaux R⁶ étant un atome d'hydrogène et le deuxième des radicaux R⁶ représentant un atome d'hydrogène ou un radical alkyle substitué ou non substitué ayant 1 à 10 atomes de carbone, en particulier un méthyle, R⁷ représentant un atome d'hydrogène, un radical alkyle substitué ou non substitué ayant 1 à 10 atomes de carbone, notamment ayant 1 à 4 atomes de carbone, et R⁸ représentant un radical de la formule (4) dans laquelle R¹⁰ représente un radical alkylène de la formule -(CH₂)ₚ, p étant un nombre entier de 1 à 6, en particulier 3, chaque R¹¹ représentant indépendamment un radical alkyle substitué ou non substitué ayant 1 à 10 atomes de carbone, notamment 1 à 4 atomes de carbone, de manière particulièrement préférée un méthyle ou éthyle, et chaque R¹² représentant indépendamment un radical alkyle substitué ou non substitué ayant 1 à 10 atomes de carbone, notamment ayant 1 à 4 atomes de carbone, de manière particulièrement préférée un méthyle ou éthyle.

9. Composition durcissable selon l'une des revendications 1 à 8, **caractérisée en ce que** l'aminosilane est un aminosilane de la formule (6)
(R¹⁵R⁷N)-R¹⁰-SiR¹¹ₒ(OR¹²)₃₋ₒ (6)
dans laquelle
o, R⁷, R¹⁰, R¹¹ et chaque R¹² ont chacun indépendamment les significations données dans la revendication 1, et
R¹⁵ représente :
un atome d'hydrogène,
un radical alkyle, alcényle ou alcynyle substitué ou non substitué.

10. Composition durcissable selon l'une des revendications 1 à 9, **caractérisée en ce que** l'aminosilane est choisi parmi le 3-aminopropyltriméthoxysilane, le 3-aminopropyltriéthoxysilane, l'aminométhyltriméthoxysilane, l'aminométhyltriéthoxysilane, le 3-(N,N-diméthylamino)propyltriméthoxysilane, le 3-(N,N-diméthylamino)propyltriéthoxysilane, le (N,N-diméthylamino)méthyltriméthoxysilane, le (N,N-diméthylamino)méthyltriéthoxysilane, la bis(triméthoxysilylpropyl)amine, la bis(3-triéthoxysilyl)propylamine et leurs mélanges.

11. Composition durcissable selon l'une des revendications 1 à 10, **caractérisée en ce que** le composé de l'étain est un composé d'organoétain choisi parmi les composés 1,3-dicarbonylés de l'étain divalent ou tétravalent, les dialkylétain-(IV)-dicarboxylates, les dialkylétain-(IV)-dicarboxylates, les dialkylétain-(IV)-dialcoxylates, les dialkylétain(IV)-oxydes, les étain-(II)-carboxylates et leurs mélanges.

12. Procédé de préparation de la composition durcissable selon l'une des revendications 1 à 11, **caractérisée en ce que** le polyorganosiloxane, comportant au moins un groupe hydroxyle lié à un atome de silicium, le silane de la formule (1), l'aminosilane, le composé de l'étain et éventuellement au moins un autre ingrédient sont mélangés ensemble.

13. Procédé selon la revendication 12, **caractérisé en ce que** le polyorganosiloxane, comportant au moins un groupe hydroxyle lié à un atome de silicium, et le silane de la formule (1) sont mélangés dans une première étape, ce mélange étant effectué en présence de la quantité totale d'aminosilane ou d'une partie de celui-ci, et éventuellement d'au moins un plastifiant, la partie restante de l'aminosilane et éventuellement d'autres ingrédients étant ajoutés dans une deuxième étape et tous les composants étant mélangés, le composé de l'étain étant ajouté dans une troisième étape et mélangé aux autres constituants.

14. Utilisation d'une composition durcissable selon l'une des revendications 1 à 11, ou une composition durcissable préparée par un procédé selon l'une des revendications 12 à 13 comme substance de collage, d'étanchéité ou de revêtement.
